# EUROPEAN PATENT SPECIFICATION

(11) **EP 4 294 363 B1**
(45) Date of publication and mention of the grant of the patent: **09.04.2025**
(21) Application number: 22704903.8
(22) Date of filing: 08.02.2022
(51) Int. Cl.: A61K 8/46, A61K 8/49, A61K 8/67, A61Q 19/08

(54) **LRAT INHIBITORS FOR TREATING SKIN AGEING**
LRAT-INHIBITOREN ZUR BEHANDLUNG DER HAUTALTERUNG
INHIBITEURS DE LRAT POUR LE TRAITEMENT DU VIEILLISSEMENT CUTANÉ

(30) Priority: 16.02.2021 EP 21157280
(43) Date of publication of application: 27.12.2023
(73) Proprietor: DSM IP Assets B.V., 6221 BE Maastricht (NL)
(72) Inventor: IMFELD, Dominik, 4303 Kaiseraugst (CH); SCHUETZ, Rolf, 4303 Kaiseraugst (CH); RAWLINGS, Anthony, Northwich, Cheshire CW98FH (GB)
(74) Representative: dsm-firmenich IP
(86) International application number: PCT/EP2022/053021
(87) International publication number: WO 2022/175136

(56) References cited:
- EP-A2- 0 739 886
- WO-A1-96/05189
- WO-A2-02/02074
- US-A- 4 049 645
- US-A- 5 518 735
- JÖRG STÜRZEBECHER ET AL: "Synthesis and Structure-Activity Relationships of Potent Thrombin Inhibitors: Piperazides of Amidinophenylalanine", JOURNAL OF MEDICINAL CHEMISTRY, AMERICAN CHEMICAL SOCIETY, vol. 19, no. 40, 12 September 1997 (1997-09-12), pages 3091 - 3099, XP002077904, ISSN: 0022-2623

## Description

The present invention relates to use of certain amidino substituted amino acid derivatives as lecithin retinol acyltransferase inhibitors (LRAT inhibitors), to cosmetic compositions comprising said amino acid derivatives as well as the use thereof for the treatment of skin ageing.

Retinoids are a class of chemical compounds that are vitamers of vitamin A or are chemically related to it and encompass inter alia retinoic acid, retinol as well as esters thereof. Retinoids have many important functions throughout the body including roles in vision, regulation of cell proliferation and differentiation, growth of bone tissue, immune function, and activation of tumor suppressor genes. Originally, used as an anti-acne treatment, Albert Kligman first identified the anti-wrinkle benefits of oral retinoids. Today, retinoids are considered as the "golden standard" for topical treatment of premature skin ageing. Tretinoin (all-trans retinoic acid) is the benchmark prescription topical therapy for improving fine facial wrinkles. Retinol as well as esters thereof are used as cosmetic agents for the prevention or treatment of skin ageing. However, the susceptibility of retinoids to degradation upon storage is still a limiting factor for their widespread use.

Retinoic acid mediates its effect via binding to its nuclear transcription factors. Two main types of nuclear retinoid receptor exist: retinoic acid receptor (RAR) which binds all trans retinoic acid (RA) and its stereoisomer 9-cis RA; and the more recently described retinoid X receptor (RXR) which binds 9-cis RA.

A common feature of these receptors is that they bind to certain regions of DNA known as hormone response elements and thereby initiate ligand dependent gene transcription. The retinoid transcription factors bind to a retinoic acid response element (RARE) in the promoter of genes composed of a six base pair sequence (AGGTCA). RAR's and RXR's are known to contain at least 3 different subtypes, alpha, beta & gamma each of which have several isoforms. The RXR's predominate in human skin, especially RXRalpha. Of the RAR's 87% are RARgamma & 13% RARalpha.

In the epidermis, retinol is metabolized to retinoic acid. However, substantial amounts of retinol are also converted to retinyl esters as storage reserves. Two enzymes are known to be responsible for the formation said retinyl esters: ARAT (acyl-CoA: retinol acyltransferase) transfers the fatty acyl group from a fatty acyl-CoA to retinol, while LRAT (lecithin: retinol acyltransferase) transfers the sn-1 fatty acyl group from phosphatidyl choline to retinol. Recent work has shown that while ARAT is mainly responsible for retinol esterification in non-proliferative, differentiated keratinocytes, LRAT is mainly responsible for retinol esterification in the basal proliferating keratinocytes (Kurlandsky et al. Cell Biology and Metabolism, Vol 271, Issue 25, 15346-15352). Thus, inhibition of LRAT allows more retinol to be available for subsequent conversion to retinoic acid making LRAT an attractive target for the development of alternative anti-ageing skin actives.

Type I and III collagens are formed in human skin in a higher proportion relative to other types and are maintained in a fixed proportion relative to one another in normal skin tissue. They are the main constituents of the dermal extracellular matrix and play a major role in skin elasticity and aspect. Collagen fibers in the dermis are composed by 80-85% of collagen I and by 10-15% of collagen III. Collagen I is the main component of collagen fibers, while collagen III is implicated mainly in their reticulation. Collagen III is a fibrillar collagen associated with collagen I and collagen V in order to form collagen fibers. However, type I and III collagen respective contents and distribution in skin vary as a function of age and therefore to quantity of both collagen I and III are key markers for skin age. In studies involving skin from donors with ages 18 up to 50, it was shown that the mean content of type I and III and type I/III collagen ratio in skin differed significantly among age groups (p < 0.05), with the lowest levels of type I, III, and the highest ratio of type I/III observed in the elderly age group. It was concluded that the amount of collagen III significantly diminishes with age. During the physiological ageing different enzymes, including collagenase, neutral protease and lysosomal cathepsins, can degrade collagens. So during skin ageing collagen I and collagen III content decrease, with a faster decrease of collagen III compared to collagen I. Retinoids are well known to stimulate the synthesis of collagen I and III in skin.

Due to significant side effects, the cosmetic use of retinoic acid is not allowed. Thus, the cosmetic industry relies on the use of retinol (ROH), retinaldehyde (RA) or retinylesters (RE). However, as all of the cosmetically acceptable retinoids, need to be metabolized to retinoic acid to mediate their effects, the benefits, that can be derived from these agents, are obviously less potent than with retinoic acid.

Thus, there is an ongoing desire in the cosmetic industry to boost the efficacy of (endogenous) retinol in order to be able to reduce the concentration thereof in topical compositions and thus to overcome stability issues associated therewith. WO 02/02074 A2 discloses skin care compositions comprising a retinoid and a combination of at least two retinoid boosters.

Surprisingly it has now been found, that certain amidino substituted amino acid derivatives are highly effective LRAT inhibitors and are in particular suitable to enhance the beneficial skin-ageing effects of retinol, reflected by an increased epidermal thickness and increased collagen levels. Furthermore, they are stable upon formulation in topical compositions and thus particularly suitable for the use in cosmetic anti-ageing products.

Various publications disclose the use of benzamidine type structures as thrombin inhibitors (WO96/05189, EP0739886, Stürzenbecher et al, J. Med. Chem 1997, Vol 40, No. 19, p. 3091-3099). US4,049,645 discloses N²naphthalenesulfonyl-L-arginine esters and amidines as antithrombotic active ingredients. US5518735 discloses certain phenylalanine derivatives as new protease inhibitors.

Thus, in a first embodiment, the present invention relates to a cosmetic use of a topical composition comprising at least one compound formula (I) or (II). 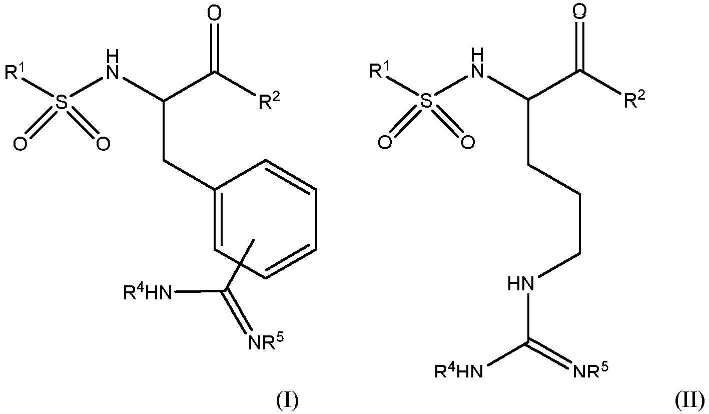 wherein
R¹ is a C₆-C₂₀alkyl group or an aryl group;
R² is OH, SH, a C₁-C₅alkoxy group, a C₁-C₅thioalkoxy group or a residue of formula (III) 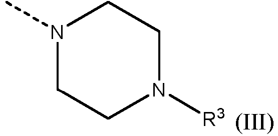 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof and a cosmetically acceptable carrier for preventing and/or treating skin ageing.

In a second embodiment, the present invention is directed to the use of a compound of formula (I) or (II) or a cosmetically acceptable salt thereof as an inhibitor for LRAT (lecithin: retinol acyltransferase), in particular for increasing the collagen level, such as in particular collagen III level in the skin and/or to increase the epidermal thickness, most in particular in aged, senescent and/or photo damaged skin.

In a third embodiment the present invention is directed to the a method to increase the effectiveness of endogenous retinol by inhibiting retinol esterification in (preferably the basal proliferating) keratinocytes (and accordingly increase the concentration of free, endogenous retinol), in particular in the treatment of skin ageing, said method comprising the step of topically applying a compound or a composition according to the present invention with all the definitions and preferences given herein, optionally together with one or more cosmetically acceptable retinoids, to the skin, and when compared to an untreated control.

As increased levels of collagen and/or an increased epidermal thickness lead to a reduction of the visible manifestations of skin ageing such as in particular of wrinkles, fine lines, sagging, and laxity, another subject matter of the invention is directed to a method for preventing and/or treating skin ageing, preferably to smoothen wrinkles and fine lines, to decrease their volume and depth, to treat skin sagging and/or to improve skin firmness said method comprising the step of topically applying a compound of formula (I) or (II) or a cosmetically acceptable salt thereof according to the present invention with all the definitions and preferences given herein to the affected area and optionally appreciating the effect.

In a further embodiment, the present invention also relates to topical skin care compositions in the form of emulsions comprising at least one compound formula (I) or (II). 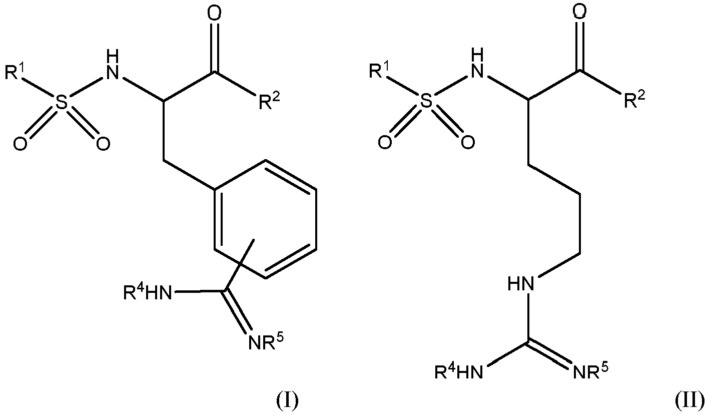 wherein
R¹ is a C₆-C₂₀alkyl group or a naphthyl group;
R² is OH, SH, a C₁-C₅alkoxy group, a C₁-C₅thioalkoxy group or a residue if formula (III) 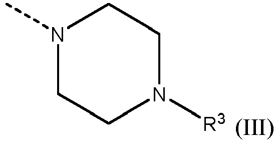 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof and a cosmetically acceptable carrier suitable for topical use as such compositions are still novel.

In addition, the present invention also relates to cosmetic compositions comprising a retinoid and at least one compound formula (I) or (II) 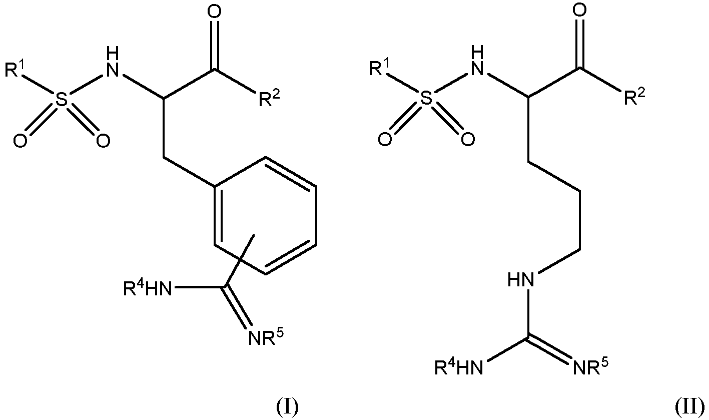 wherein
R¹ is a C₆-C₂₀alkyl group or an aryl group;
R² is OH, SH, a C₁-C₅alkoxy group, a C₁-C₅thioalkoxy group or a residue if formula (III) 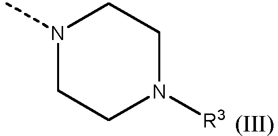 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof and a cosmetically acceptable carrier, as such compositions are also novel.

It is well understood, that all compositions according to the present invention are intended for topical application, which is to be understood as the external application to keratinous substances, such as in particular the skin. Particularly preferred cosmetic compositions are topical compositions.

For sake of clarity, some terms as been used in the present document are defined as follows:
The term 'cosmetic composition' as used herein refers to compositions, which are topically applied and which are used to treat, care for or improve the appearance of (i.e. beautify) the skin.

The term 'prevention of skin ageing' as used herein refers to a prophylactic use to reduce the risk to develop visible manifestations of skin ageing such as in particular wrinkles, fine lines, sagging, and laxity.

The term 'treatment of skin ageing' as used herein refers to a reduction of the visible manifestations of skin ageing such as in particular wrinkles, fine lines, sagging, and laxity.

The term 'cosmetically acceptable carrier' as used herein refers to all carriers and/or excipients and/or diluents conventionally used in cosmetic compositions such as in particular in skin care preparations.

It is well understood by a person skilled in the art, that the amidino residue (R⁴HN-C=NR⁵) in formula (I) is either in ortho (2), meta (3) or para (4) position of the phenyl residue, preferably, in all embodiments of the present invention the amidino residue is either in meta or in para position.

In the present document, a "Cx-y" refers to a group comprising x to y carbon atoms, i.e., for example, a C₁₋₃₋alkyl group is an alkyl group comprising 1 to 3 carbon atoms.

The term 'Cₓ-C_{y}alkyl group' is used herein refers to monovalent straight or branched hydrocarbyl groups having from X to Y carbon atoms. Depending on the definition of 'x' and 'y' suitable Cₓ-C_{y}alkyl groups according to the present invention encompass methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, 2,4,4-trimethylpentyl and 3,5,5-trimethylhexyl groups, n-octyl, n-nonyl, n-decyl, n-undecyl, n-dodecyl, myristyl, palmityl, stearyl and eicosyl. Preferred in all embodiments of the present invention are straight chain (linear) Cₓ-C_{y}alkyl groups.

The term 'aryl group' as used herein refers to monovalent, aromatic, cyclic hydrocarbyl groups, such as phenyl or naphthyl (e.g. 1-naphthyl or 2-naphthyl). In general, the aryl group may be a monocyclic or polycyclic fused ring aromatic group. Preferred aryl groups in all embodiments of the present invention are C₆₋₁₄aryl, more preferably C₆₋₁₀aryl, most preferably naphtyl, such as in particular 2-naphtyl.

The term 'C₁-C₅alkoxy group' is used herein to refer an -O-C₁-C₅alkyl group - with all the definitions and preferences for Cₓ-C_{y}alkyl as given above, such as in particular methoxy (-OMe) or ethoxy (-OEt). Most preferred in all embodiments of the present invention is methoxy.

The term 'C₁-C₂₀alkylsulfonyl group' is used herein to refer to a -S(O₂)C₁-C₁₅alkyl group - with all the definitions and preferences for Cₓ-C_{y}alkyl as given above. Preferred in all embodiments of the present invention is a -S(O₂)C₁-C₁₂alkyl group, more preferred a -S(O₂)C₁-C₁₀alkyl group, most preferred -S(O₂)CH₃ (-S(O₂)Me) or -S(O₂)C₇H₁₄CH₃ (-S(O₂)Octyl).

The term 'thioCₓ-C_{y}alkoxyl group' is used herein to refer to a -S-Cₓ-C_{y}alkyl group - with all the definitions and preferences for Cₓ-C_{y}alkyl as given above. Preferred in all embodiments of the present invention is a -S-C₁-C₅alkyl group, more preferred a -S-C₁-C₃alkyl group, most preferred -SCH₃ (-SMe).

The term 'C₁-C₅acyl' refers to a -C(O)C₁-C₅alkyl group - with all the definitions and preferences for Cₓ-C_{y}alkyl as given above such as e.g. acetyl, propionyl, n-butyryl, isobutyryl and pentanoyl. Preferred C₁-C₅acyl groups in all embodiments according to the present invention are C₁-C₃acyl groups. Most preferred in all embodiments of the present invention is acetyl.

The term 'alkylene group' is used herein to refer to divalent saturated, linear or branched hydrocarbon groups. Exemplary alkylene groups include -CH₂-, -(CH₂)₂-,-(CH₂)₃-, -C(CH₃)₂CH₂- and -C(CH₃)H-CH₂-,. Preferred alkylene groups are saturated linear ones having from 1 to 4 carbon atoms, more preferably from 2 to 3 carbon atoms, most preferably 2 carbon atoms (i.e. ethylene (-(CH₂)₂-)). Furthermore it is even more preferred if none of the carbon atoms is replaced by a heteroatom as defined herein.

The term 'alkenylene group' is used herein to refer to divalent linear or branched hydrocarbon groups, comprising at least one double bond. Exemplary alkenylene groups include -CH=CH-, -CH=CHCH₂-, and -CH₂CH=CH-. The most preferred alkenylene group is ethenylene (i.e. -CH=CH-).

Preferably, in all embodiments of the present invention R¹ is linear C₈-C₁₅alkyl group or an C₆₋₁₀aryl group, more preferably a linear C₁₀-C₁₂alkyl group or naphth-2-yl, most preferably dodecyl.

Preferably, in all embodiments of the present invention R² is OH, a linear C₁-C₃alkoxy group or a residue of formula (III) wherein R³ is selected from a linear C₁-C₁₂alkylsulfonyl group or a linear C₁-C₃acyl group, most preferably R² is OH, OMe or a residue of formula (III) wherein R³ is a linear C₁-C₁₀alkylsulfonyl group.

Preferably, in all embodiments of the present invention R⁴ is H and R⁵ is H, NH₂ or a C₁₋₃alkyl group or R⁴ and R⁵ together are a linear C₁₋₄alkylene or a linear C₁₋₄alkenylene group, most preferably R⁵ is H, NH₂ or methyl or R⁴ and R⁵together are ethylene.

Particular advantageous compounds of formula (I) or (II), respectively cosmetically acceptable salts thereof in all embodiments of the present invention are the ones, wherein
R¹ is a linear C₈-C₁₅alkyl group or an C₆₋₁₀aryl group;
R² is OH, a linear C₁-C₃alkoxy group, or a residue of formula (III) 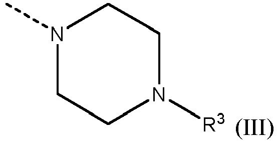 wherein the dotted line represents the connection to the carbonyl group and R³ is selected from a linear C₁-C₁₂alkylsulfonyl group or a linear C₁-C₃acyl group;
R⁴ is H;
R⁵ is H, NH₂ or a C₁₋₃ alkyl group;
or R⁴ and R⁵ together are a linear C₁₋₄alkylene or a linear C₁₋₄alkenylene group.

Even more advantageous compounds of formula (I) or (II), respectively cosmetically acceptable salts thereof in all embodiments of the present invention are the ones, wherein
R¹ is a linear C₁₀-C₁₂alkyl group or a napht-2-yl group;
R² is OH, a methoxy group, or a residue of formula (III) 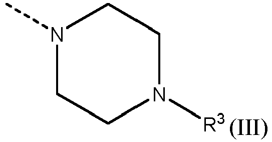 wherein the dotted line represents the connection to the carbonyl group and R³ is selected from a linear C₁-C₁₀alkylsulfonyl group;
R⁴ is H;
R⁵ is H, NH₂ or methyl;
or R⁴ and R⁵ together are ethylene.

Particularly preferred compounds of formula (I) in all embodiments of the present invention are compounds of formula (la) or (Ib) 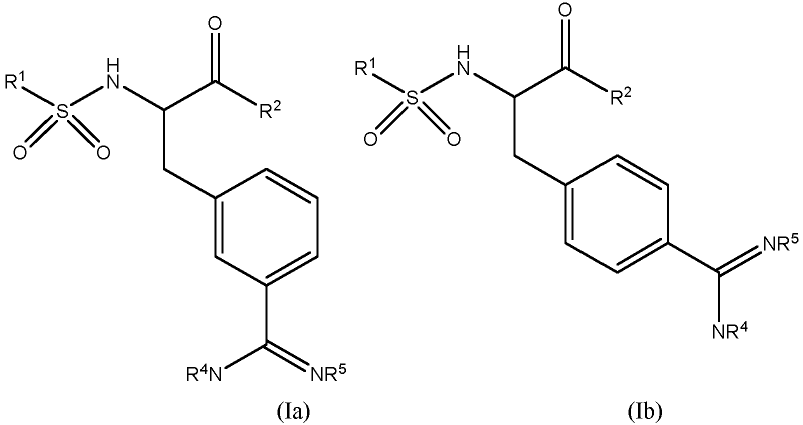 wherein
R¹ is a linear C₁₀-C₁₂alkyl group or a napht-2-yl group;
R² is OH or a residue of formula (III) 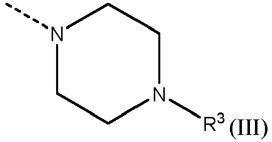 wherein the dotted line represents the connection to the carbonyl group and R³ is selected from a linear C₁-C₁₂alkylsulfonyl group, preferably a linear C₁-C₁₀alkylsulfonyl group;
R⁴ is H;
R⁵ is H, methyl or NH₂;
or R⁴ and R⁵ together are ethylene;
or a cosmetically acceptable salt thereof.

Particularly preferred compounds of formula (II) in all embodiments of the present invention are compounds of formula (IIa) 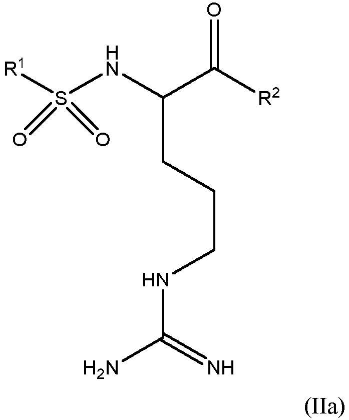 wherein
R¹ is a linear C₁₀-C₁₂alkyl group;
R² is OH, OMe, or a residue of formula (III) 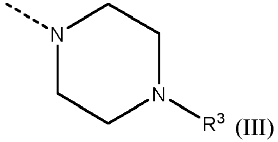 wherein the dotted line represents the connection to the carbonyl group and R³ is selected from a C₁-C₁₂alkylsulfonyl, preferably a C₁-C₅alkylsulfonyl, most preferably methylsulfonyl;
R⁴ is H;
R⁵ is H, NH₂ or a C₁₋₅ alkyl group, preferably H, NH₂ or a methyl;
or R⁴ and R⁵ together are an alkylene or an alkenylene, in which alkylene or alkyenylene optionally one of the carbon atoms may be replaced by O or NH, preferably ethylene;
or a cosmetically acceptable salt thereof.

Most preferred in all embodiments according to the present invention are the compounds listed in table 1 as well as the respective chloride, iodide or acetate salts thereof.

| Code* | | Structure |
|---|---|---|
| DODS-(D,L)-F(3AMD)-Pzd(N-SO₂Me) | (A) | 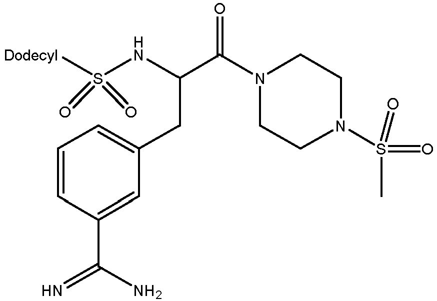 |
| DODS-(L)-F(3AMD)-Pzd(N-SO₂Me) | (B) | 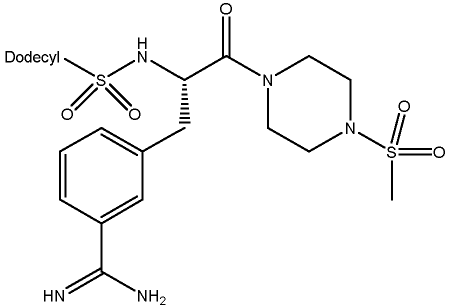 |
| DODS-(L)-F(3MAMD)-Pzd(N-SO₂Me) | (C) | 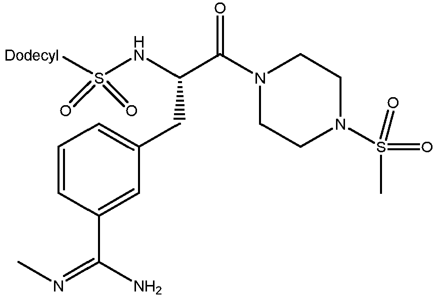 |
| DECS-(D,L)-F(3AMD)-Pzd(N-SO₂Me) | (D) | 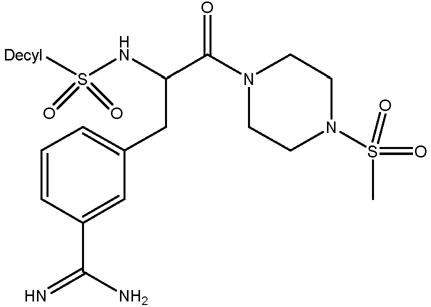 |
| DODS-(L)-F(3AZO)-Pzd(N-SO₂Me) | (E) | 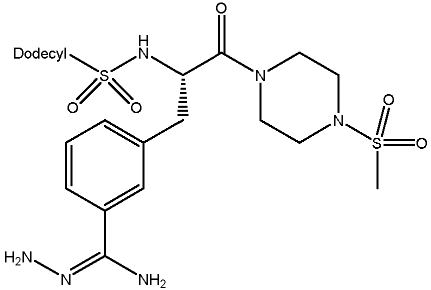 |
| DODS-(L)-F(3IMD)-Pzd(N-SO₂Me) | (F) | 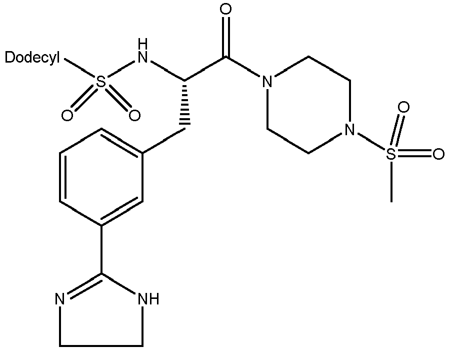 |
| DODS-(L)-F(3AMD)-OH | (G) | 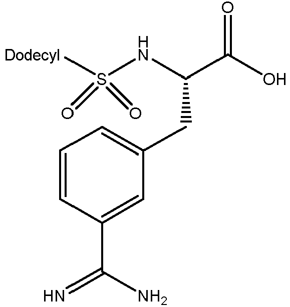 |
| DODS-(L)-F(3AMD)-Pzd(N-SO₂Oct) | (H) | 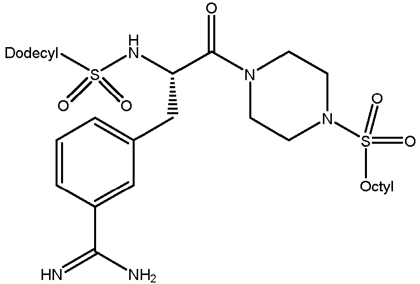 |
| DODS-(L)-F(4AMD)-Pzd(N-SO₂Me) | (I) | 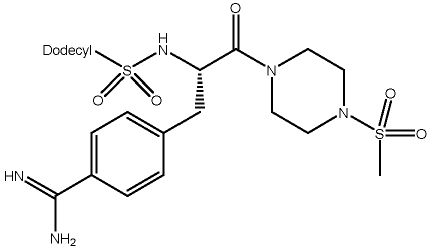 |
| DODS-(L)-F(3AMD)-Pzd(N-H) | (J) | 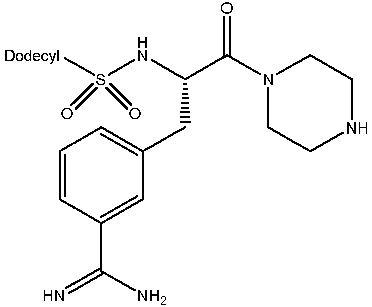 |
| OCTS-(D,L)-F(3AMD)-Pzd(N-SO₂Me) | (K) | 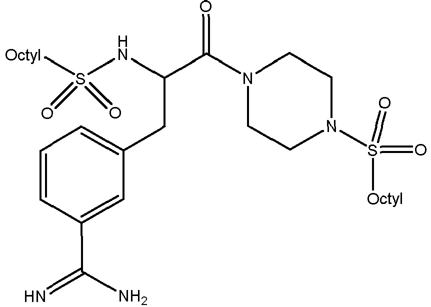 |
| HEDS-(D,L)-F(3AMD)-Pzd(N-SO₂Me) | (L) | 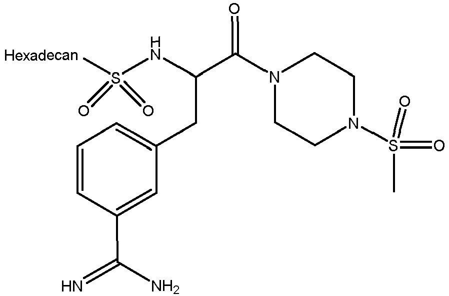 |
| 2NAPS-(D,L)-F(3AMD)-Pzd(N-SO₂OCt) | (M) | 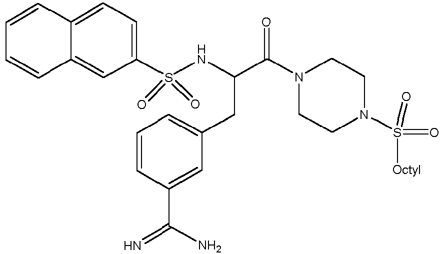 |
| 2NAPS-(L)-F(3AMD)-Pzd(N-Ac) | (N) | 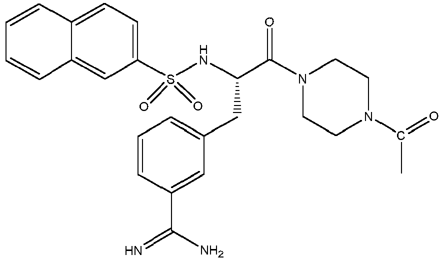 |
| DODS-Arg-OH | (O) | 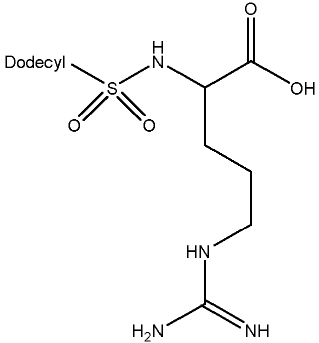 |
| DODS-Arg-OMe | (P) | 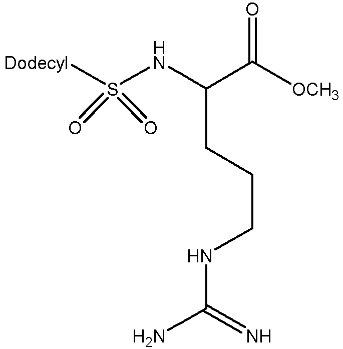 |
| DODS-Arg-Pzd(N-SO₂Me) | (Q) | 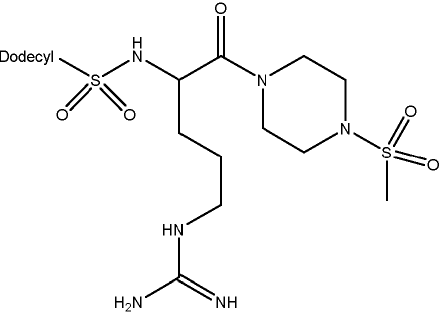 |

| | | |
|---|---|---|
| *Abbreviations: Ac: Acetyl; AMD: amidino; Arg: Arginin; AZO: Aminoamidino; DODS: dodecansulfonyl; DECS: decansulfonyl; F: Phenylalanin; HEDS: n-hexadecansulfonyl; IMD: 4,5-dihydro-1*H*-imidazol-2-yl; MAMD: methylamidino; Me: Methyl; 2NAPS: 2-naphthylsulfonyl, Oct: Octyl; OCTS: octansulfonyl; Pzd, piperazinylamido, SO₂Me: methylsulfonyl; SO₂Oct: octylsulfinyl(sulfoxide) | | |

The compounds according to the present invention can be prepared by standard methods in the art such as e.g. by reacting the respective amino acid (e.g. arginine) with the respective sulfonyl chloride (e.g. 1-dodecylsulfonyl chloride or 2-naphthalenesulfonyl chloride) in the presence of a base and/or as outlined in WO96/05189A.

It is well understood, that the present invention encompasses the compounds of formula (I) or (II) as optically pure isomers such as e.g. as pure enantiomers or stereoisomers as well as mixtures of different isomers such as e.g. as racemates, or mixtures of diastereoisomers.

It is furthermore well understood, that the respective amidino groups encompasses the respective cis/ trans as well as tautomeric forms as outlined below:

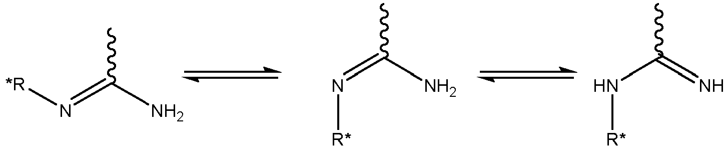

The term 'or a cosmetically acceptable salt thereof' refers to compounds of formula (I) or (II) with all the definitions and preferences as given herein in the form of an acid addition salt such as in the form of a triflate, a chloride, an iodide, an acetate or a trifluoroacetate salt.

Most preferred, in all embodiments of the present invention, are the compounds of formula (I) or (II) as such or in the form of their acetates, chlorides or iodides. Such salts are easily prepared by a person skilled in the art.

It is well understood, that the at least one compound of formula (I) or (II) or the respective salts thereof are generally applied to skin in the form of compositions suitable for the topical application to human skin (topical compositions) such as in particular in the form of cosmetic compositions.

The amount of the compound of formula (I) or (II) or the respective salts thereof in the compositions according to the present invention can easily be adjusted by a person skilled in the art in order to achieve the desired beneficial effect. Preferably, the amount of the compound of formula (I) or (II) or the respective salts thereof is at least 1 ppm based on the total weight of the composition. In all embodiments of the present invention the amount of the compound of formula (I) or (II) or the respective salts thereof is preferably selected in the range from 0.0001 to 5.0 wt.-%, more preferably in the range from 0.001 to 1.0 wt.-%, most preferably in the range from 0.01 to 0.1 wt.-% based on the total weight of the composition. Further preferred ranges encompass 0.0001 to 1.0 wt.-%, 0.0001 to 0.5, 0.0001 to 0.1 wt.-%, 0.001 to 1.0 wt.-%, 0.001 to 0.5, 0.001 to 0.1 wt.-%, 0.01 to 1.0 wt.-%, 0.01 to 0.5, 0.01 to 0.1 wt.-%.

As the compositions according to the invention are intended for cosmetic application, it is well understood that they comprise a cosmetically acceptable carrier i.e. a physiologically acceptable medium, i.e. a medium compatible with keratinous substances, such as the skin, mucous membranes, and keratinous fibres.

Particular advantageous compositions in all embodiments of the present invention are skin care compositions.

Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well-known to one of ordinary skilled in the art, and can include one or more compatible liquid(s) or solid filler diluent(s), excipient(s), additive(s) or vehicle(s) which are suitable for application to skin. The exact amount of carrier will depend upon the level of the compound of formula (I) or (II) respectively the salts thereof and any other optional ingredients that one of ordinary skilled in the art would classify as distinct from the carrier (e.g., other active components). The compositions of the present invention preferably comprise from about 75% to about 99.999%, more preferably from about 85% to about 99.99%, still more preferably from 90% to about 99%, and most preferably, from about 93% to about 98%, by weight of the composition, of a carrier.

The exact amount of carrier will depend upon the actual level of the compound of formula (I) or (II) respectively a salt thereof and any other optional ingredients that one of ordinary skill in the art would classify as distinct from the carrier (e.g., other active ingredients).

In a particular advantageous embodiment, the carrier consists furthermore of at least 30 wt. %, more preferably of at least 40 wt.-%, most preferably of at least 45 wt.-% of water, such as in particular of 50 to 90 wt.-% of water.

The compositions of the present invention can be formulated into a wide variety of product types, including creams, waxes, pastes, lotions, milks, mousses, gels, oils, tonics, and sprays. Preferably the compounds of formula (I) or (II) respectively the corresponding salts thereof are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If the compositions of the present invention are formulated as an aerosol and applied to the skin as a spray-on product, a propellant is added to the composition.

The compositions according to the present invention can be prepared by conventional methods in the art such as e.g. by admixing a compound of formula (I) or (II) or the corresponding salts thereof with all the definitions and preferences given herein with the cosmetically acceptable carrier.

The compositions of the invention (including the carrier) may comprise further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/or sequestering agents, essential oils, skin sensates, astringents, pigments or any other ingredients usually formulated into such compositions.

Preferred compositions according to the present invention include a retinoid, preferably in an amount selected in the range from 0.001 to 10 wt.-%, preferably from about 0.005 to about 0.5.-%, most preferably from about 0.03 to about 0.3 wt.-%, based on the total weight of the composition of a retinoid.

Suitable retinoids include retinol as well as retinyl esters such as in particular retinyl linoleate, retinyl palmitate, retinyl oleate, retinyl propionate, retinyl laurate, retinyl octanoate, retinyl phenylbutyrate, retinyl alkyl carbonate, retinoxytrimethylsilane, (all trans)-retinal or its acetals, or methoxy PEG-12 retinamide and retinyl acetate, retinol being most preferred. Also suitable are all kind of encapsulated retinoids such as for example Cyclasphere^{®} retinol.

Further preferred compositions according to the present invention include one or more hydroxystearic acid, such as in particular 12-hydroxystearic acid, 10-hydroxystearic acid and 9-hydroxystearic acid, preferably in an amount selected in the range from 0.05 to 5 wt.-%, preferably from about 0.1 to 2.5.-%, most preferably from about 0.25 to 1.5 wt.-%, based on the total weight of the composition of a hydroxystearic acid.

In accordance with the present invention, the compositions according to the invention may also comprise further cosmetically active ingredients conventionally used in cosmetic compositions. Exemplary active ingredients encompass further skin lightening agents; UV-filters, agents for the treatment of hyperpigmentation; agents for the prevention or reduction of inflammation; firming, moisturizing, soothing, and/or energizing agents as well as agents to improve elasticity and skin barrier.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/or sequestering agents, essential oils, skin sensates, astringents, pigments or any other ingredients usually formulated into such compositions.

If nothing else is stated, the excipients, additives, diluents, etc. mentioned in the following are suitable for sunscreen compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Exemplary compositions according to the invention are skin care preparations, decorative preparations, and functional preparations, skin care preparations and functional preparations being particularly preferred.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

The compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the composition according to the present invention is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearyl sulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%, based on the total weight of the composition.

The compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The amount of the composition according to the present invention to be applied to the skin is not critical and can easily be adjusted by a person skilled in the art. Preferably the amount is selected in the range of 0.1 to 3 mg/cm² skin, such as preferably in the range of 0.1 to 2 mg/cm² skin and most preferably in the range of 0.5 to 2 mg/cm² skin.

In accordance with the invention is the use of the compounds of formula (I) and (II) respectively the corresponding cosmetically acceptable salts thereof with all the definitions and preferences as given herein or the composition comprising said compounds according to the present invention as an agent for stimulating the synthesis of collagen in skin, thickening the epidermis, conditioning and smoothening the skin and/or preventing or reducing the appearance of wrinkled or aged skin.

Finally, a subject-matter of the invention is a method of mimicking the effect of topical retinoic acid, said method comprising applying a compound of formula (I) or (II) respectively a cosmetically acceptable salt thereof or the composition according to the present invention comprising said compound to an area of the skin in need thereof.

The following examples are provided to further illustrate the compositions and effects of the present invention. These examples are illustrative only and are not intended to limit the scope of the invention in any way.

### Experimental Part

### 1. LRAT inhibition assay

The following buffer was prepared and stored at 4°C: 0.05 M potassium phosphate, 2.5 mM dithioerythritol, pH 7.0 (PO₄/DTE). On the day of the assay, 1 mg BSA per mL of buffer was added to give a P0₄/DTE/BSA working buffer. Retinol substrate (Fluka, 1 mM) was prepared in acetonitrile (Riedel-de-Haen, Chromasolv) and stored in amber bottles under nitrogen gas at -20°C. Solutions of 4 mM dilauroyl phosphatidyl choline in ethanol were prepared and stored at -20°C. Inhibitors were prepared as 10 mM stock solutions according their solubilities in H₂O, ethanol, acetonitrile, DMSO or acetic acid. The quenching solution was prepared using pure ethanol containing 50 µg/mL butylhydroxytoluene (Aldrich, BHT) A pentane solution containing 50 µg/mL BHT was used for the extractions.

To a 7.4 mL (2 dram) glass vial, the following were added in order: P0₄/DTT/BSA buffer to give a total volume of 500 µL, 5 µL acyl donor (dilauroyl phosphatidyl choline), 5 µL inhibitor or solvent blank (10 mM stock or further dilutions) followed by 2 µg recombinant LRAT enzyme (Abnova, H00009227-PO1). The mixture was incubated for 5 min. at 37°C to equilibrate the reaction temperature and then 5 µL of a 1 mM retinol solution was added. The vials were capped, vortexed for 5 seconds and incubated for 60 minutes at 37°C. The reaction was quenched by adding 0.5 mL of a ethanol/BHT quenching solution. The retinoids were extracted by adding 1 mL pentane/BHT, vortexing the tubes for several seconds and centrifuging the tubes at 4000 rpm in a bench-top centrifuge for 5 min. to quickly separate the layers. The upper pentane layer was removed into a clean vial, and the aqueous layer re-extracted twice with another 1 mL pentane/BHT, as described above. The organic layers were combined, and the pentane evaporated by drying at 37°C in a speed-vac centrifuge (Eppendorf). The dried residue was stored at -20°C and directly analyzed by HPLC. The amount of retinyl laurate was quantified for LRAT activity by integration of the HPLC or UPLC signal as described below. Note that the incubation solution contains 40 µM acyl donor, 100 uM inhibitor, 10 uM retinol, approximately 2 µg LRAT protein, and 0.05 M P0₄/ , 2.5 mM DTT, 1 mg/mL BSA at pH 7. All steps subsequent to the addition of retinol were done in the dark or under amber lights.

Two HPLC protocols were used for retinoid analysis. The separation of retinol and retinol esters was performed with a reverse-phase analytical column (Waters Symmetry (R), 75x4.6mm, 3.5 µm; Eluent A: 0.07% trifluoroacetic acid TFA in water, B: 0.07% TFA in acetonitrile (AcN), linear gradient from 1% B to 99% B in 6min, 24min on 99% B) or ACQUITY UPLC BEH Phenyl 1.7 µm 2.1x50mm (0.02% TFA in water and eluent B, 0.02% TFA in AcN with a gradient of 2% to 99% B in 0.9 min, 99% B for 1 min, from 99% to 2% B in 0.1 min and flow rate 0.5 ml/min at 40°C. The eluate was monitored with a PDA detector for absorbance at 325nm. Quantification of the retinol and retinylester peak was based on references retinoic acid and retinyl acetate (Fluka). The inhibition [%] was calculated relative to the product yield from the non-inhibited control reaction. The results are outlined in table 2.

**Table 2: LRAT inhibition**

| **Inhibitor** | **concentration [µm]** | **Inhibition [%]** |
|---|---|---|
| DODS-(D,L)-F(3AMD)-Pzd(N-SO₂Me) x HCl | 100 | 94.3 |
| DODS-(L)-F(3AMD)-Pzd(N-SO₂Me) x HOAc | 100 | 97.0 |
| DODS-(L)-F(3AMD)-Pzd(N-SO₂Me) x HCl | 10 | 91.4 |
| DODS-(L)-F(3AMD)-Pzd(N-SO₂Me) x HOAc | 100 | 92.6 |
| DODS-(L)-F(3MAMD)-Pzd(N-SO₂Me) x HI | 100 | 94.1 |
| DECS-(D,L)-F(3AMD)-Pzd(N-SO₂Me) x·HOAc | 100 | 91.5 |
| DODS-(L)-F(3AZO)-Pzd(N-SO₂Me) x·HOAc | 100 | 98.1 |
| DODS-(L)-F(3IMD)-Pzd(N-SO₂Me) x HOAc | 100 | 98.7 |
| DODS-(L)-F(3AMD)-OH x·HOAc | 100 | 96.0 |
| DODS-(L)-F(3AMD)-Pzd(N-SO₂Oct) x·HOAc | 100 | 100.0 |
| DODS-(L)-F(4AMD)-Pzd(N-SO₂Me) x HOAc | 100 | 96.7 |
| DODS-(L)-F(3AMD)-Pzd(N-H) | 100 | 44.6 |
| OCTS-(D,L)-F(3AMD)-Pzd(N-SO₂Oct) | 100 | 89.9 |
| HEDS-(D,L)-F(3AMD)-Pzd(N-SO₂Me) | 100 | 39.3 |
| 2NAPS-(D,L)-F(3AMD)-Pzd(N-SO₂Oct) x HCl | 100 | 94.4 |
| 2NAPS-(L)-F(3AMD)-Pzd(N-Ac) | 100 | 38.7 |
| DODS-Arg-OH | 100 | 84.6 |
| DODS-Arg-OMe x HOAc | 100 | 94.3 |
| DODS-Arg-Pzd(N-SO₂Me) x HOAc | 100 | 96.0 |

| *Negative Controls* | | |
|---|---|---|
| H-(D,L)-F(3AMD)-Pzd(N-SO₂Me) x 2 HCl | 100 | 6.8 |
| DODS-(L)-F(4NH₂)-Pzd(N-SO₂Me) | 100 | 0 |

### 2. Evaluation of the anti-ageing effects of two different LRAT-inhibitors on living human skin explants

### Method:

### Preparation of skin explants:

On an abdoplasty coming from a 45-year-old Caucasian woman with a type II phototype, 51 circular skin explants of 11 ± 1 mm in diameter were prepared. The explants were kept for survival in BEM culture medium (BIO-EC's Explants Medium, Longjumeau, France) at 37°C in a humid, 5 % CO2 atmosphere

### Application of test samples:

Each morning on day 0, 1, 4 and 6 the test products (except retinol) were applied topically on the basis of 2 µl/ explants (2 mg/cm2) of the concerned skin explants and spread using a small spatula. Each evening on day 0, 1, 4, 6 the retinol was applied topically on the basis of 2 µl/ explants (2 mg/cm2) of the concerned skin explants and spread using a small spatula. The vehicle V (DMSO 100%) was applied topically (same protocol as for products) in the morning and in the evening on day0, 1, 4, 6. The culture medium was refreshed on day1, 4 and 6.

### Sampling:

On day 0, 3 explants from the batch at day 0 were collected and cut in two parts. Half was fixed in buffered formalin solution and half was frozen at -80°C. On day 8, 3 explants from each experimental condition were collected and processed in the same way as on day 0.

### Histological processing:

After fixation for 24 hours in buffered formalin, the samples were dehydrated and impregnated in paraffin using a Leica PEARL dehydration automat. The samples were embedded using a Leica EG 1160 embedding station. 5-µm-thick sections were made using a Leica RM 2125 Minot-type microtome, and the sections were mounted on Superfrost^{®} histological glass slides. The frozen samples were cut at 7-µm thickness with a Leica CM 3050 cryostat. The sections were then mounted on silanized glass slides Superfrost^{®} Plus. The microscopical observations were realized using a Leica DMLB or Olympus BX43 microscope. Pictures were digitized with a numeric DP72 Olympus camera with cellSens storing software.

### Viability assessment and epidermal thickness:

The cell viability of the epidermal and dermal structures was controlled on formalin-fixed paraffin-embedded (FFPE) skin sections after to Masson's trichrome staining, Goldner variant. The cell viability was assessed by microscopical observation.

### Collagen III immunostaining:

Collagen III immunostaining was realized on frozen skin sections with an anti- collagen III polyclonal antibody (SBA, ref. 1330-01), diluted at 1:200 in PBS-BSA 0.3% and incubated for 1 hour at room temperature with a biotin/ streptavidin amplifying system and revealed with VIP (Vector laboratories, Ref. SK-4600), a substrate of peroxidase giving a violet staining once oxidized. The staining was assessed by microscopical observation

### Results

The viability assessment of the skin explants after 8 days in culture showed good results for all experimental conditions. The results of the epidermal thickness and the modulation of collagen III protein are shown in table 3.

The test compounds LRAT-IA and B are:
LRAT-IA = DECS-(L)-F(3AMD)-Pzd(N-SO₂Me),
LRAT-IB = DODS-(L)-F(3AMD)-Pzd(N-SO₂Me)

The control tissues showed both at day0 and day8 the same staining intensity.

**Table 3: change of epidermal thickness/ collagen III staining compared to control (vehicle only) on day 8**

| Retinol concentration | LRAT Inhibitor | Epidermis thickness | Collagen III level |
|---|---|---|---|
| Retinol 0.05% | - | + | + |
| - | LRAT-IA 0.005% | (+) | 0 |
| - | LRAT-IA 0.01% | + | 0 |
| - | LRAT-IA 0.1% | n.a. | ++ |
| Retinol 0.05% | LRAT-IA 0.005% | + | + |
| Retinol 0.05% | LRAT-IA 0.01% | + | ++ |
| Retinol 0.05% | LRAT-IA 0.1% | + | +++ |
| - | LRAT-IB 0.005% | (+) | + |
| - | LRAT-IB 0.01% | (+) | ++ |
| - | LRAT-IB 0.1% | (+) | +++ |
| Retinol 0.05% | LRAT-IB 0.005% | (+) | +++ |
| Retinol 0.05% | LRAT-IB 0.01% | + | ++ |
| Retinol 0.05% | LRAT-IB 0.1% | + | ++++ |

| | | | |
|---|---|---|---|
| Legend: 0 = unchanged, (+) = very slight increased, + = slight increased ++ = moderate increased +++ = fairly clear increased ++++ = clear increased | | | |

As can be retrieved from table 3, the LRAT inhibitors alone lead to an increase in the epidermal thickness and/or the collagen III level, which effect is particularly pronounced in the presence of retinol respectively for the dodecyl derivative.

### 3. Cosmetic composition

Table 4 outlines exemplary O/W emulsions.

**Table 4 Anti ageing cream**

| **Ingredients** | **INCI Name** | **% (wt)** | **% (wt)** | **% (wt)** | **% (wt)** |
|---|---|---|---|---|---|
| WATER DEM. | AQUA | Ad. 100 | Ad. 100 | Ad. 100 | Ad. 100 |
| Edeta BD | DISODIUM EDTA, AQUA | 0.05 | 0.05 | 0.05 | 0.05 |
| Keltrol CG-T | XANTHAN GUM | 0.20 | 0.20 | 0.20 | 0.20 |
| Zemea Propanediol | PROPANEDIOL | 10.0 | 10.0 | 10.0 | 10.0 |
| | | | | | |
| Olivem 1000 | CETEARYL OLIVATE, SORBITAN OLIVATE | 3.00 | 3.00 | 3.00 | 3.00 |
| Eumulgin SML 20 | POLYSORBATE 20 | 0.50 | 0.50 | 0.50 | 0.50 |
| Cutina FS 45 | STEARIC ACID, PALMITIC ACID | 1.00 | 1.00 | 1.00 | 1.00 |
| Isopropyl Myristate | ISOPROPYL MYRISTATE | 4.00 | 4.00 | 4.00 | 4.00 |
| Isostearyl Isostearate | ISOSTEARYL ISOSTEARATE | 4.00 | 4.00 | 4.00 | 4.00 |
| Cetiol OE | DICAPRYLYL ETHER | 4.00 | 4.00 | 4.00 | 4.00 |
| Euxyl PE 9010 | PHENOXYETHANOL, ETHYLHEXYLGLYCERIN | 1.00 | 1.00 | 1.00 | 1.00 |
| Xiameter PMX-200 Sil Fluid 100 CS | DIMETHICONE | 1.00 | 1.00 | 1.00 | 1.00 |
| Sepinov EMT 10 | HYDROXYETHYL ACRYLATE/SODIUM ACRYLOYLDIMETHYL TAURATE COPOLYMER | 0.50 | 0.50 | 0.50 | 0.50 |
| | | | | | |
| DODS-(L)-F(3AMD)-Pzd(N-SO2Me) | n.a. | 0.15 | 0.05 | 0.10 | 0.10 |
| RETINOL GS 50 | RETINOL, POLYSORBATE 20, BHT, BHA | - | 0.25 | 0.10 | 0.10 |
| 10-Hydroxystearic acid | Hydroxystearic acid | - | - | | 0.5 |
| WATER DEM. | AQUA | 3.70 | 3.70 | 3.70 | 3.70 |
| | | | | | |
| Sodium hydroxide (10% ) | SODIUM HYDROXIDE, AQUA | 0.03 | 0.03 | 0.03 | 0.03 |

## Claims

1. Cosmetic use of a topical composition comprising at least one compound formula (I) or (II). 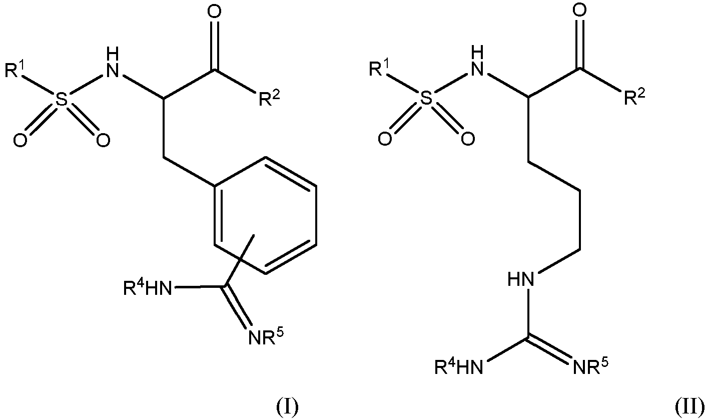 wherein
R¹ is a C₆-C₂₀alkyl group or an aryl group;
R² is OH, SH, a C₁-C₅alkoxy group, a C₁-C₅thioalkoxy group or a residue if formula (III) 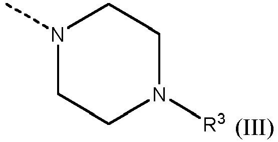 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof for preventing and/or treating skin ageing.

2. The cosmetic use according to claim 1, wherein R¹ is linear C₈-C₁₅alkyl group or an C₆₋₁₀aryl group, more preferably a linear C₁₀-C₁₂alkyl group or naphth-2-yl, most preferably dodecyl.

3. The cosmetic use according to claim 1 or 2, wherein R² is OH, a linear C₁-C₃alkoxy group or a residue of formula (III) wherein R³ is selected from a linear C₁-C₁₂alkylsulfonyl group or a linear C₁-C₃acyl group, most preferably R² is OH, OMe or a residue of formula (III) wherein R³ is a linear C₁-C₁₂alkylsulfonyl group.

4. The cosmetic use according to any one of the preceding claims, wherein R⁴ is H and R⁵ is H, NH₂ or a C₁₋₃ alkyl group or R⁴ and R⁵ together are a linear C₁₋₄alkylene or a linear C₁₋₄alkenylene group, most preferably R⁵ is H, NH₂ or methyl or R⁴ and R⁵ together are ethylene.

5. The cosmetic use according to any one of the preceding claims, wherein the compound of formula (I) is a compound of formula (la) or (Ib) 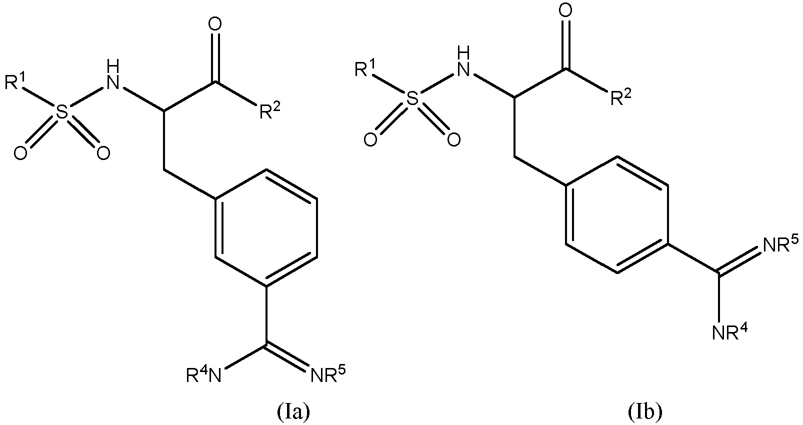 wherein
R¹ is a C₁₀-C₁₂alkyl group or a napht-2-yl group;
R² is OH or a residue of formula (III) 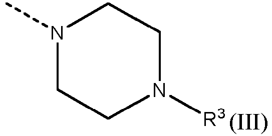 wherein the dotted line represents the connection to the carbonyl group and R³ is selected from a linear C₁-C₁₂alkylsulfonyl group;
R⁴ is H;
R⁵ is H, Methyl or NH₂;
or R⁴ and R⁵ together are ethylene,
or a cosmetically acceptable salt thereof.

6. The cosmetic use according to any one of the preceding claims, wherein the compound of formula (II) is a compound of formula (IIa) 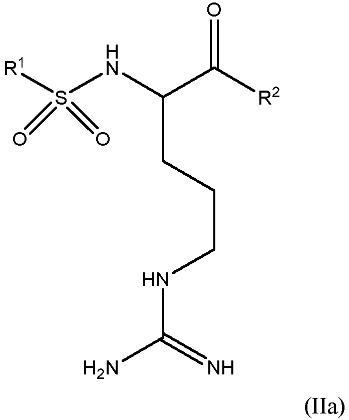 wherein
R¹ is a C₁₀-C₁₂alkyl group;
R² is OH, OMe, or a residue of formula (III) 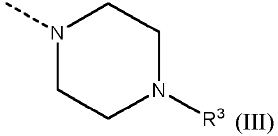
wherein the dotted line represents the connection to the carbonyl group and
R³ is selected from a C₁-C₁₂alkylsulfonyl group, preferably methylsulfonyl
R⁴ is H;
R⁵ is H, NH₂ or a C₁₋₅ alkyl group;
or R⁴ and R⁵ together are an alkylene or an alkenylene, in which optionally one of the carbon atoms may be replaced by O or NH;
or a cosmetically acceptable salt thereof.

7. The cosmetic use according to any one of the preceding claims, wherein the compound of formula (I) or (II) is a compound of formula
| | |
|---|---|
| (A) | 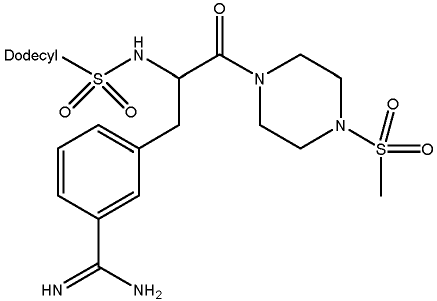 |
| (B) | 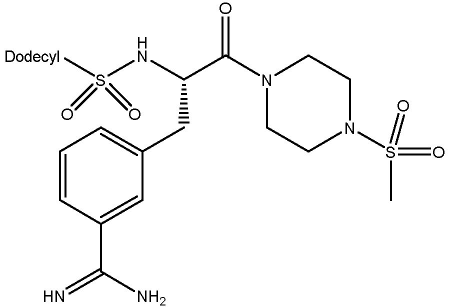 |
| (C) | 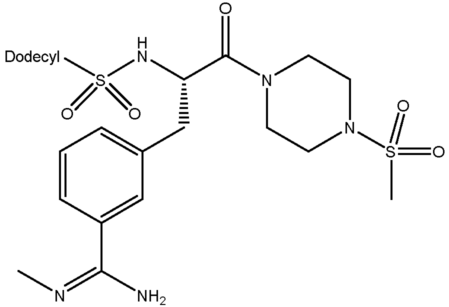 |
| (D) | 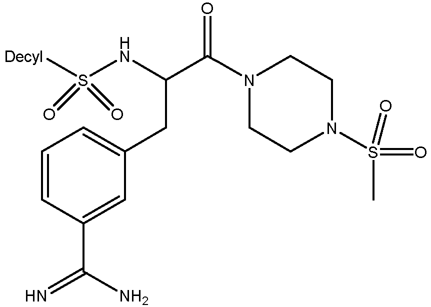 |
| (E) | 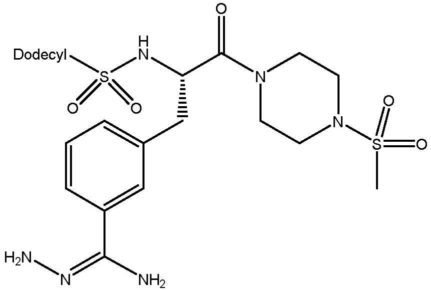 |
| (F) | 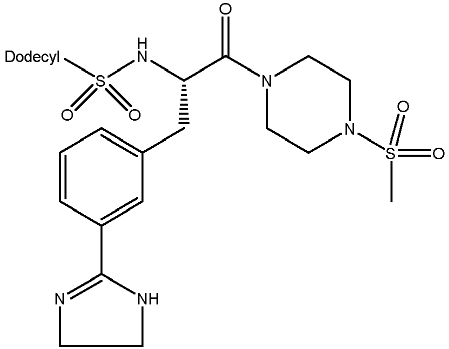 |
| (G) | 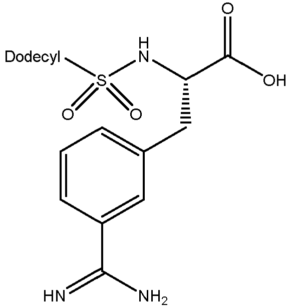 |
| (H) | 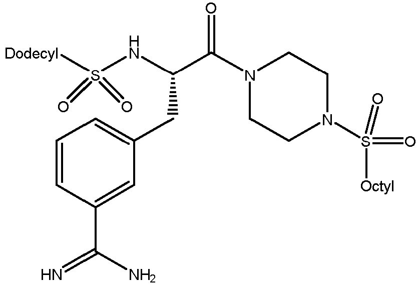 |
| (I) | 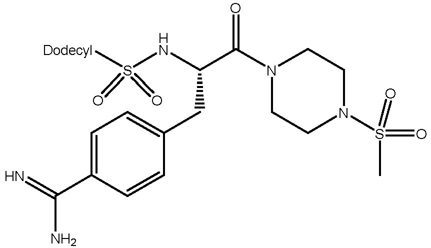 |
| (J) | 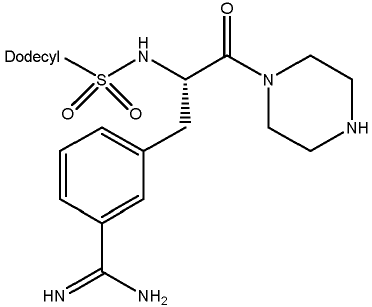 |
| (K) | 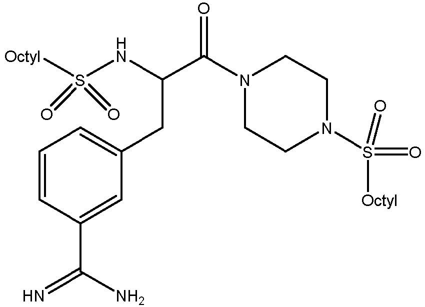 |
| (L) | 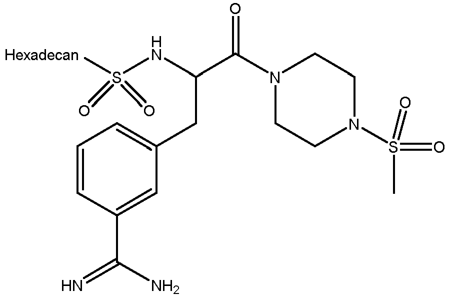 |
| (M) | 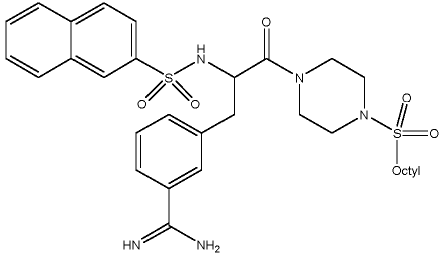 |
| (N) | 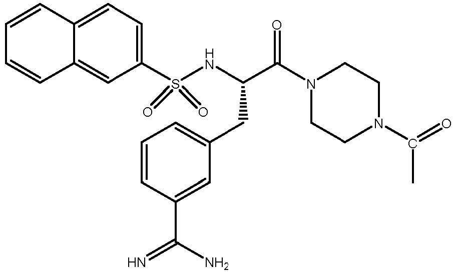 |
| (O) | 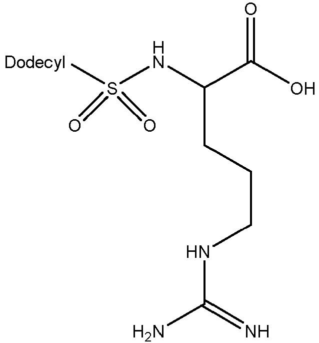 |
| (P) | 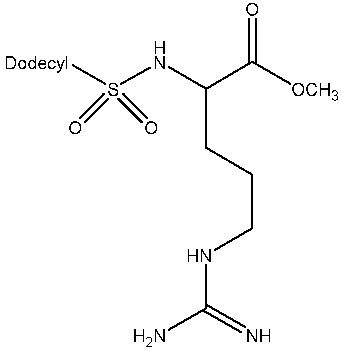 |
| (Q) | 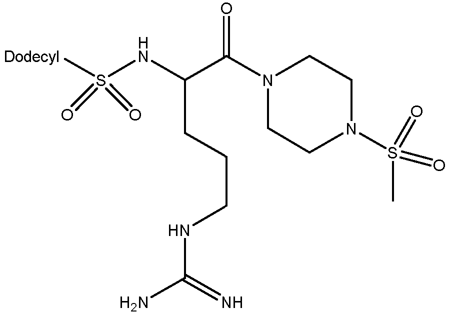 |
or a cosmetically acceptable salt thereof.

8. The cosmetic use according to any one of the preceding claims, wherein the amount of the compound of formula (I) or (II) or the respective salts thereof in the composition is selected in the range from 0.0001 to 5.0 wt.-%, preferably in the range from 0.001 to 1.0 wt.-%, most preferably in the range from 0.01 to 0.1 wt.-%, based on the total weight of the composition

9. The cosmetic use according to any one of the preceding claims, wherein the composition further comprises a retinoid, preferably retinol.

10. The cosmetic use according to claim 9, wherein the amount of the retinoid is selected in the range from 0.001 to 10 wt.-%, preferably from about 0.005 to 0.5 wt.-%, most preferably from about 0.03 to 0.3 wt.-%, based on the total weight of the composition.

11. Cosmetic use of a compound of formula (I) or (II) or a cosmetically acceptable salt thereof as defined in anyone of claim 1 to 7 as a LRAT (lecithin: retinol acyltransferase) inhibitor.

12. Method to increase the effectiveness of endogenous retinol by inhibiting retinol esterification in keratinocytes, in particular in the treatment of skin ageing, said method comprising the step of topically applying a compound of formula (I) or (II) or a cosmetically acceptable salt thereof as defined in anyone of claims 1 to 7 to the skin of a person in need thereof, wherein the effectiveness is assessed compared to an untreated control.

13. Method for preventing and/or treating skin ageing, preferably to smoothen wrinkles and fine lines, to decrease their volume and depth, to treat skin sagging and/or to improve skin firmness in a person in need thereof, said method comprising the step of topically applying a compound as defined in anyone of claim 1 to 7 to the affected area.

14. A topical skin care composition in the form of an emulsion comprising at least one compound formula (I) or (II). 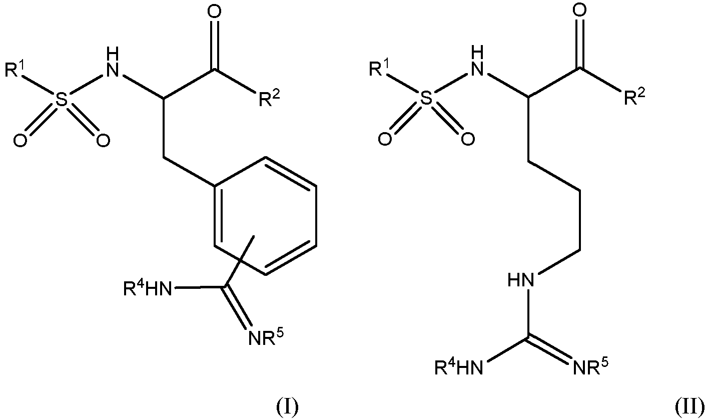 wherein
R¹ is a C₆-C₂₀alkyl group or a naphthyl group;
R² is OH, SH, a C₁-C₅alkoxy group, a C₁-C₅thioalkoxy group or a residue if formula (III) 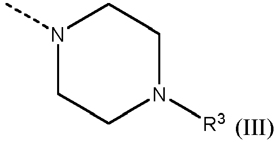 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof and a cosmetically acceptable carrier.

15. A cosmetic composition comprising a retinoid and at least one compound formula (I) or (II). 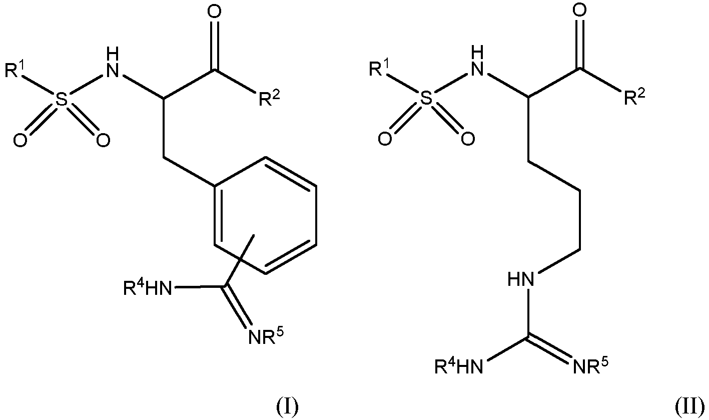 wherein
R¹ is a C₆-C₂₀alkyl group or an aryl group;
R² is OH, SH, a C₁-C₅alkoxy group, a C₁-C₅thioalkoxy group or a residue if formula (III) 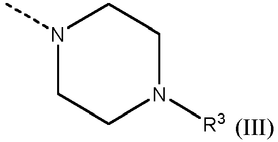 wherein the dotted line represents the connection to the carbonyl group and R³ is selected from H, a C₁-C₁₅alkylsulfonyl group or a C₁-C₅acyl group;
R⁴ is H;
R⁵ is H, NH₂ or a C₁₋₅ alkyl group;
or R⁴ and R⁵ together are an alkylene or an alkenylene group, in which optionally one of the carbon atoms may be replaced by O or NH;
or a cosmetically acceptable salt thereof and a cosmetically acceptable carrier.

## Patentansprüche

1. Kosmetische Verwendung einer topischen Zusammensetzung, die mindestens eine Verbindung der Formel (I) oder (II) umfasst. 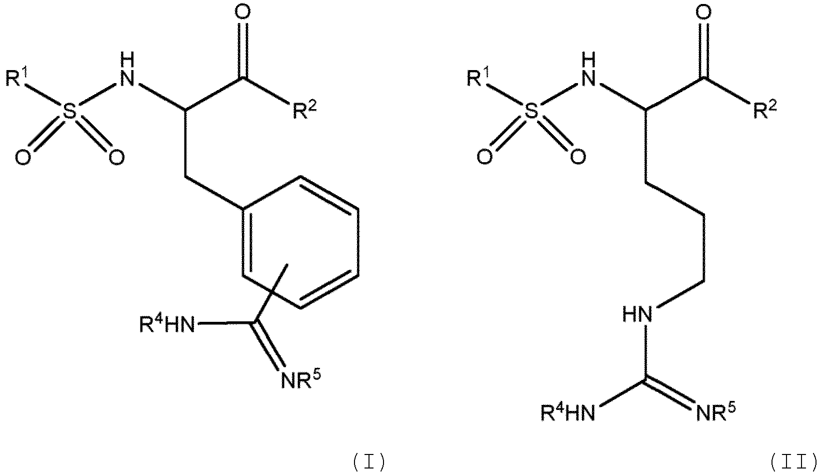 wobei
R¹ eine C₆-C₂₀-Alkylgruppe oder eine Arylgruppe ist;
R² OH, SH, eine C₁-C₅-Alkoxygruppe, eine C₁-C₅-Thioalkoxygruppe oder ein Rest der Formel (III) ist 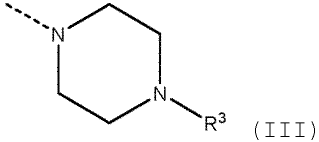
wobei die gestrichelte Linie die Verbindung mit der Carbonylgruppe darstellt und R³ aus H, einer C₁-C₁₅-Alkylsulfonylgruppe oder einer C₁-C₅-Acylgruppe ausgewählt ist;
R⁴ H ist;
R⁵ H, NH₂ oder eine C₁₋₅Alkylgruppe ist;
oder R⁴ und R⁵ zusammen ein Alkylen oder eine Alkenylengruppe sind, in der gegebenenfalls eines der Kohlenstoffatome durch O oder NH ersetzt sein kann;
oder ein kosmetisch annehmbares Salz davon zum Verhindern und/oder Behandeln von Hautalterung.

2. Kosmetische Verwendung nach Anspruch 1, wobei R¹ eine lineare C₈-C₁₅-Alkylgruppe oder eine C₆₋₁₀-Arylgruppe ist, besonders bevorzugt eine lineare C₁₀-C₁₂-Alkylgruppe oder Naphth-2-yl ist, ganz besonders bevorzugt Dodecyl ist.

3. Kosmetische Verwendung nach Anspruch 1 oder 2, wobei R² OH, eine lineare C₁-C₃-Alkoxygruppe oder ein Rest der Formel (III) ist, wobei R³ aus einer linearen C₁-C₁₂-Alkylsulfonylgruppe oder einer linearen C₁-C₃-Acylgruppe ausgewählt ist, ganz besonders bevorzugt R² OH, OMe oder ein Rest der Formel (III) ist, wobei R³ eine lineare C₁-C₁₂-Alkylsulfonylgruppe ist.

4. Kosmetische Verwendung nach einem der vorstehenden Ansprüche, wobei R⁴ H ist und R⁵ H, NH₂ oder eine C₁₋₃-Alkylgruppe ist oder R⁴ und R⁵ zusammen ein lineares C₁₋₄-Alkylen oder eine lineare C₁₋₄-Alkenylengruppe sind, ganz besonders bevorzugt R⁵ H, NH₂ oder Methyl ist oder R⁴ und R⁵ zusammen Ethylen sind.

5. Kosmetische Verwendung nach einem der vorstehenden Ansprüche, wobei die Verbindung der Formel (I) eine Verbindung der Formel (Ia) oder (Ib) ist 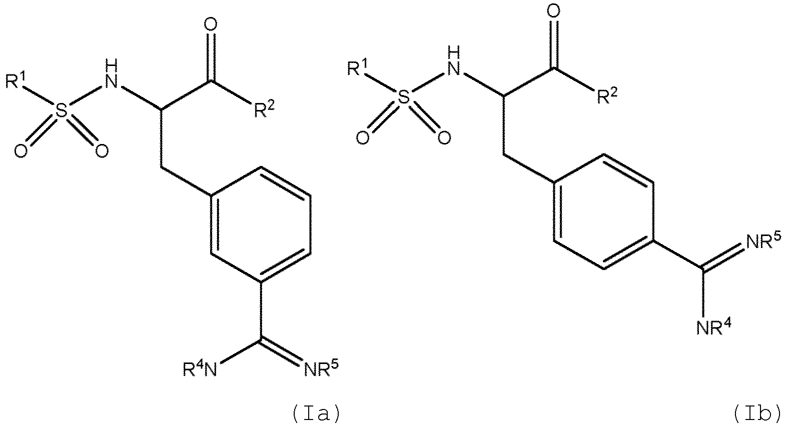 wobei
R¹ für eine C₁-C₁₂-Alkylgruppe oder eine Naphth-2-ylgruppe steht;
R² OH oder ein Rest der Formel (III) ist 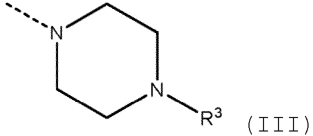
wobei die gestrichelte Linie die Verbindung mit der Carbonylgruppe darstellt und R³ aus einer linearen C₁-C₁₂-Alkylsulfonylgruppe ausgewählt ist;
R⁴ H ist;
R⁵ H, Methyl oder NH₂ ist;
oder R⁴ und R⁵ zusammen Ethylen sind,
oder ein kosmetisch annehmbares Salz davon.

6. Kosmetische Verwendung nach einem der vorstehenden Ansprüche, wobei die Verbindung der Formel (II) eine Verbindung der Formel (IIa) ist: 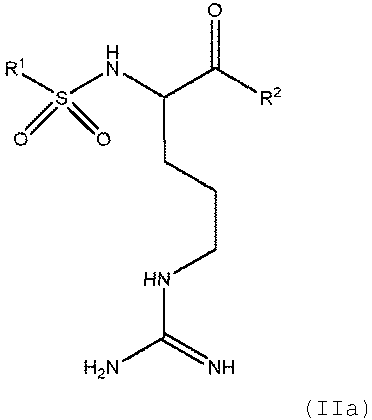 wobei
R¹ eine C₁₀-C₁₂-Alkylgruppe ist;
R² OH, OMe oder ein Rest der Formel (III) ist 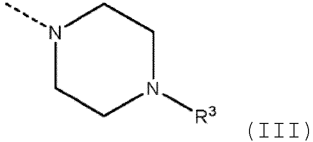
wobei die gestrichelte Linie die Verbindung mit der Carbonylgruppe darstellt und R³ aus einer C₁-C₁₂-Alkylsulfonylgruppe ausgewählt ist, vorzugsweise Methylsulfonyl;
R⁴ H ist;
R⁵ H, NH₂ oder eine C₁₋₅-Alkylgruppe ist;
oder R⁴ und R⁵ zusammen ein Alkylen oder ein Alkenylen sind, in dem gegebenenfalls eines der Kohlenstoffatome durch O oder NH ersetzt sein kann;
oder ein kosmetisch annehmbares Salz davon.

7. Kosmetische Verwendung nach einem der vorstehenden Ansprüche, wobei die Verbindung der Formel (I) oder (II) eine Verbindung der folgenden Formeln ist:
| | |
|---|---|
| (A) | 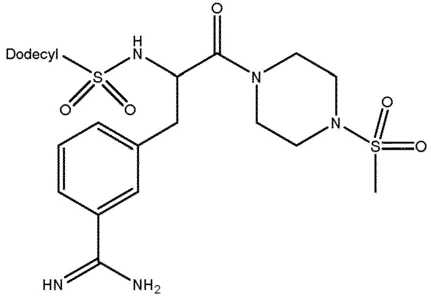 |
| (B) | 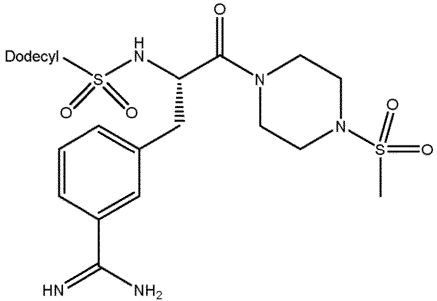 |
| (C) | 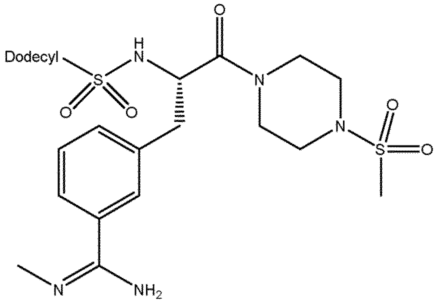 |
| (D) | 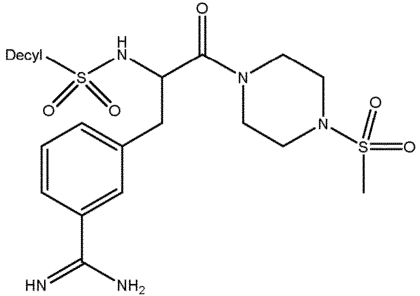 |
| (E) | 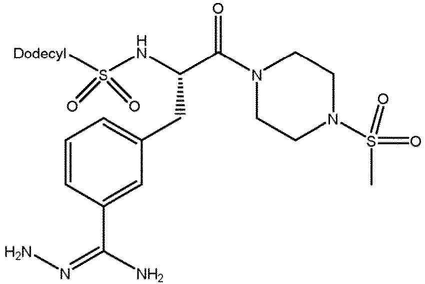 |
| (F) | 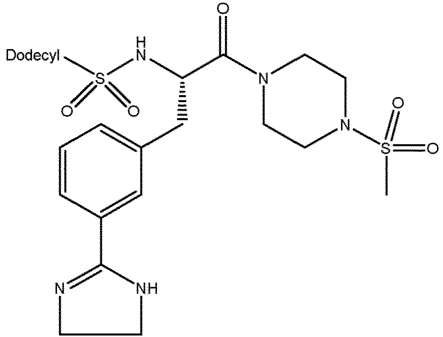 |
| (G) | 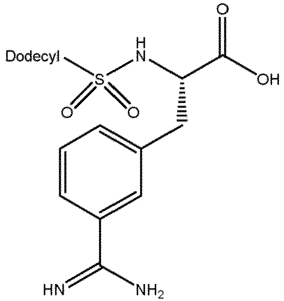 |
| (H) | 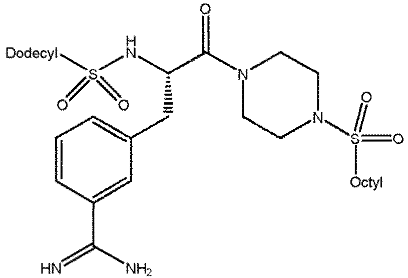 |
| (I) | 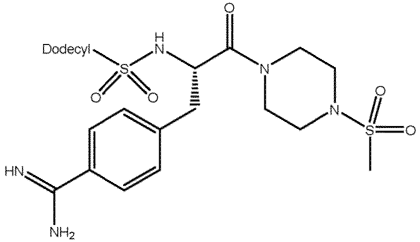 |
| (J) | 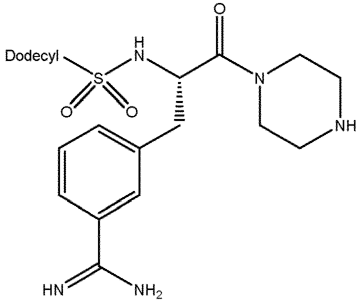 |
| (K) | 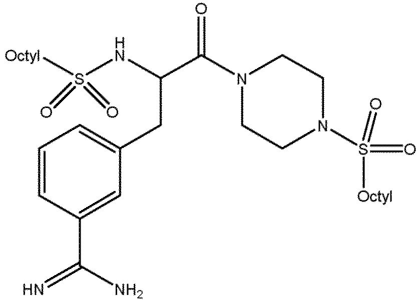 |
| (L) | 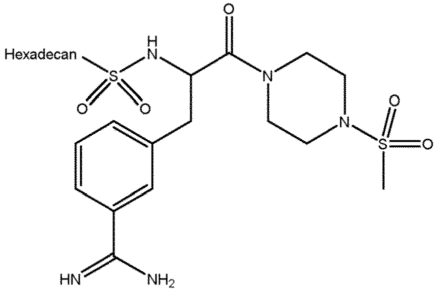 |
| (M) | 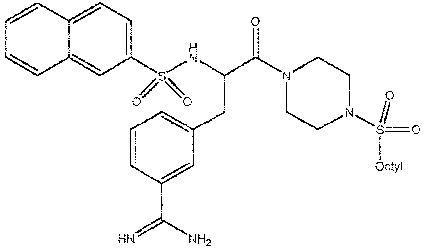 |
| (N) | 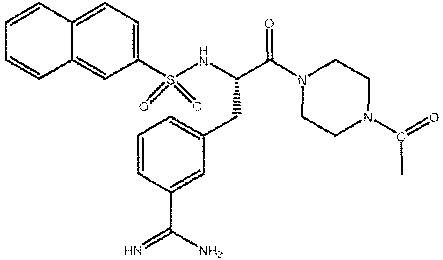 |
| (O) | 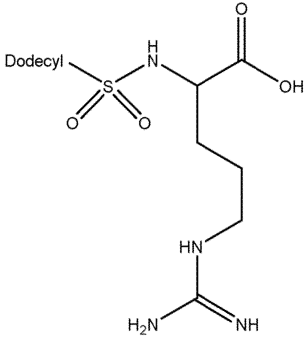 |
| (P) | 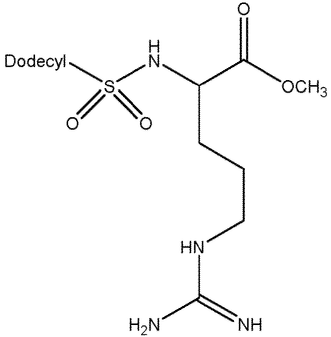 |
| (Q) | 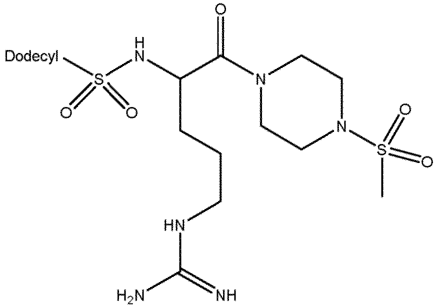 |
oder ein kosmetisch annehmbares Salz davon.

8. Kosmetische Verwendung nach einem der vorstehenden Ansprüche, wobei die Menge der Verbindung der Formel (I) oder (II) oder der jeweiligen Salze davon in der Zusammensetzung im Bereich von 0,0001 bis 5,0 Gew.-%, vorzugsweise im Bereich von 0,001 bis 1,0 Gew.-%, ganz besonders bevorzugt im Bereich von 0,01 bis 0,1 Gew.-%, bezogen auf das Gesamtgewicht der Zusammensetzung, ausgewählt ist.

9. Kosmetische Verwendung nach einem der vorstehenden Ansprüche, wobei die Zusammensetzung ferner ein Retinoid, vorzugsweise Retinol, umfasst.

10. Kosmetische Verwendung nach Anspruch 9, wobei die Menge des Retinoids im Bereich von 0,001 bis 10 Gew.-%, vorzugsweise von etwa 0,005 bis 0,5 Gew.-%, ganz besonders bevorzugt von etwa 0,03 bis 0,3 Gew.-%, bezogen auf das Gesamtgewicht der Zusammensetzung, ausgewählt ist.

11. Kosmetische Verwendung einer Verbindung der Formel (I) oder (II) oder eines kosmetisch annehmbaren Salzes davon nach einem der Ansprüche 1 bis 7 als LRAT-Inhibitor (LRAT - Lecithin-Retinol-Acyltransferase).

12. Verfahren zum Erhöhen der Wirksamkeit von endogenem Retinol durch Hemmen der Retinolveresterung in Keratinozyten, insbesondere bei der Behandlung von Hautalterung, wobei das Verfahren den Schritt des topischen Auftragens einer Verbindung der Formel (I) oder (II) oder eines kosmetisch annehmbaren Salzes davon nach einem der Ansprüche 1 bis 7 auf die Haut einer Person, die dessen bedarf, umfasst, wobei die Wirksamkeit im Vergleich zu einer unbehandelten Kontrollgruppe beurteilt wird.

13. Verfahren zum Verhindern und/oder Behandeln von Hautalterung, vorzugsweise zum Glätten von Falten und feinen Fältchen, um deren Volumen und Tiefe zu verringern, um Hauterschlaffung zu behandeln und/oder um die Hautfestigkeit bei einer Person, die dessen bedarf, zu verbessern, wobei das Verfahren den Schritt des topischen Auftragens einer Verbindung nach einem der Ansprüche 1 bis 7 auf den betroffenen Bereich umfasst.

14. Topische Hautpflegezusammensetzung in Form einer Emulsion, umfassend mindestens eine Verbindung der Formel (I) oder (II): 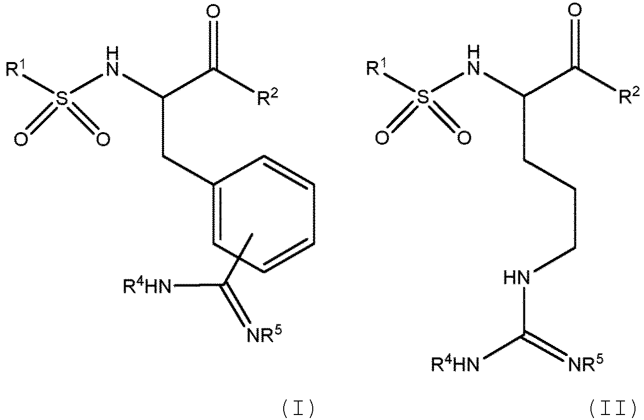 wobei
R¹ eine C₆-C₂₀-Alkylgruppe oder eine Naphthylgruppe ist;
R² OH, SH, eine C₁-C₅-Alkoxygruppe, eine C₁-C₅-Thioalkoxygruppe oder ein Rest der Formel (III) ist 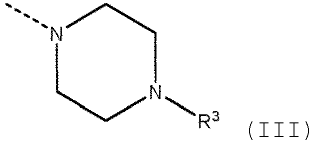
wobei die gestrichelte Linie die Verbindung mit der Carbonylgruppe darstellt und R³ aus H, einer C₁-C₁₅-Alkylsulfonylgruppe oder einer C₁-C₅-Acylgruppe ausgewählt ist;
R⁴ H ist;
R⁵ H, NH₂ oder eine C₁₋₅Alkylgruppe ist;
oder R⁴ und R⁵ zusammen ein Alkylen oder eine Alkenylengruppe sind, in der gegebenenfalls eines der Kohlenstoffatome durch O oder NH ersetzt sein kann;
oder ein kosmetisch annehmbares Salz davon und ein kosmetisch annehmbarer Träger.

15. Kosmetische Zusammensetzung, umfassend ein Retinoid und mindestens eine Verbindung der Formel (I) oder (II). 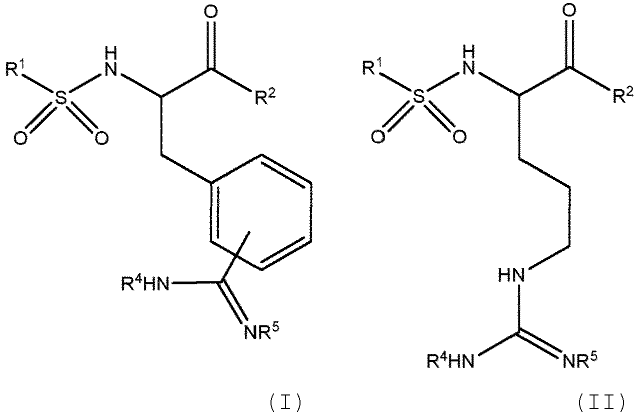 wobei
R¹ eine C₆-C₂₀-Alkylgruppe oder eine Arylgruppe ist;
R² OH, SH, eine C₁-C₅-Alkoxygruppe, eine C₁-C₅-Thioalkoxygruppe oder ein Rest der Formel (III) ist 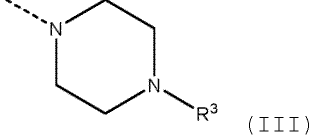
wobei die gestrichelte Linie die Verbindung mit der Carbonylgruppe darstellt und R³ aus H, einer C₁-C₁₅-Alkylsulfonylgruppe oder einer C₁-C₅-Acylgruppe ausgewählt ist;
R⁴ H ist;
R⁵ H, NH₂ oder eine C₁₋₅₋Alkylgruppe ist;
oder R⁴ und R⁵ zusammen ein Alkylen oder eine Alkenylengruppe sind, in der gegebenenfalls eines der Kohlenstoffatome durch O oder NH ersetzt sein kann;
oder ein kosmetisch annehmbares Salz davon und ein kosmetisch annehmbarer Träger.

## Revendications

1. Utilisation cosmétique d'une composition topique comprenant au moins un composé de formule (I) ou (II) 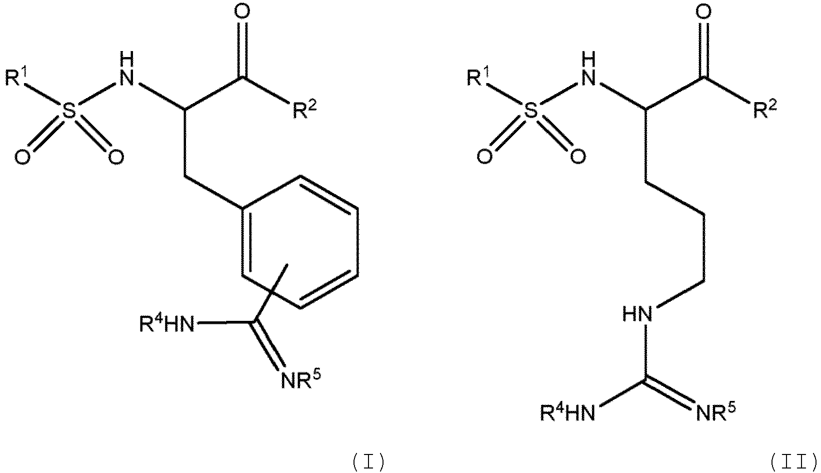 dans laquelle
R¹ est un groupe C₆-C₂₀alkyle ou un groupe aryle ;
R² est OH, SH, un groupe C₁-C₅alcoxy, un groupe C₁-C₅thioalcoxy ou un radical de formule (III) 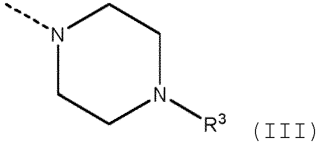
dans laquelle la ligne en pointillés représente la connexion au groupe carbonyle et R³ est choisi parmi H, un groupe C₁-C₁₅alkylsulfonyle ou un groupe C₁-C₅acyle ;
R⁴ est H ;
R⁵ est H, NH₂ ou un groupe C₁₋₅ alkyle ;
ou R⁴ et R⁵ conjointement sont un groupe alkylène ou un groupe alcénylène, dans lequel éventuellement l'un des atomes de carbone peut être remplacé par O ou NH ;
ou un sel acceptable sur le plan cosmétique correspondant pour la prévention et/ou le traitement du vieillissement cutané.

2. Utilisation cosmétique selon la revendication 1, dans laquelle R¹ est un groupe C₈-C₁₅alkyle linéaire ou un groupe C₆₋₁₀aryle, plus préférablement un groupe C₁₀-C₁₂alkyle linéaire ou napht-2-yle, le plus préférablement dodécyle.

3. Utilisation cosmétique selon la revendication 1 ou 2, dans laquelle R² est OH, un groupe C₁-C₃alcoxy linéaire ou un radical de formule (III), dans laquelle R³ est choisi parmi un groupe C₁-C₁₂alkylsulfonyle linéaire ou un groupe C₁-C₃acyle linéaire, le plus préférablement R² est OH, OMe ou un radical de formule (III), dans laquelle R³ est un groupe C₁-C₁₂alkylsulfonyle linéaire.

4. Utilisation cosmétique selon l'une quelconque des revendications précédentes, dans laquelle R⁴ est H et R⁵ est H, NH₂ ou un groupe C₁₋₃ alkyle ou R⁴ et R⁵ conjointement sont un groupe C₁₋₄alkylène linéaire ou un groupe C₁₋₄alcénylène linéaire, le plus préférablement R⁵ est H, NH₂ ou méthyle ou R⁴ et R⁵ conjointement sont éthylène.

5. Utilisation cosmétique selon l'une quelconque des revendications précédentes, dans laquelle le composé de formule (I) est un composé de formule (Ia) ou (Ib) 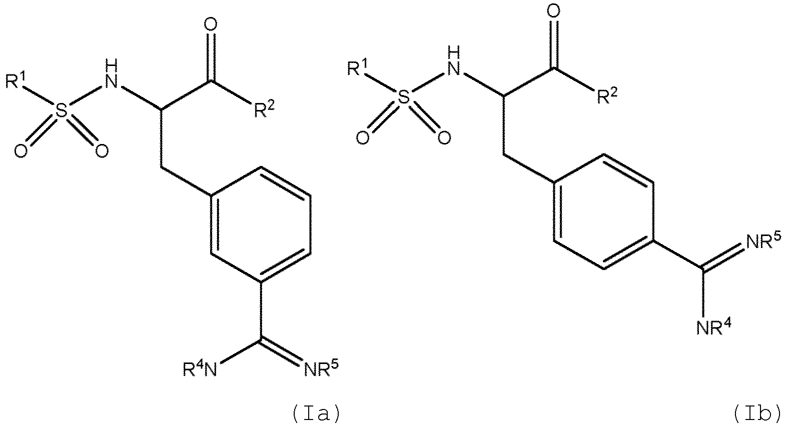 dans laquelle
R¹ est un groupe C₁₀-C₁₂alkyle ou un groupe napht-2-yle ;
R² est OH ou un radical de formule (III) 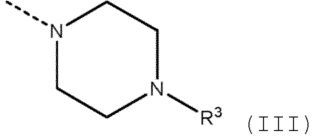
dans laquelle la ligne en pointillés représente la connexion au groupe carbonyle et R³ est choisi parmi un groupe C₁-C₁₂alkylsulfonyle ;
R⁴ est H ;
R⁵ est H, méthyle ou NH₂ ;
ou R⁴ et R⁵ conjointement sont éthylène,
ou un sel acceptable sur le plan cosmétique correspondant.

6. Utilisation cosmétique selon l'une quelconque des revendications précédentes, dans laquelle le composé de formule (II) est un composé de formule (IIa) 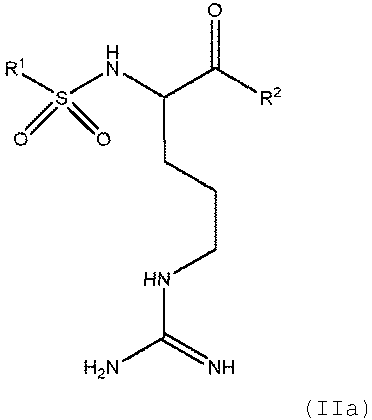 dans laquelle
R¹ est un groupe C₁₀-C₁₂alkyle ;
R² est OH, OMe, ou un radical de formule (III) 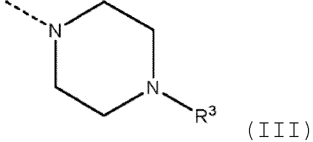
dans laquelle la ligne en pointillés représente la connexion au groupe carbonyle et R³ est choisi parmi un groupe C₁-C₁₂alkylsulfonyle, préférablement méthylsulfonyle
R⁴ est H ;
R⁵ est H, NH₂ ou un groupe C₁₋₅ alkyle ;
ou R⁴ et R⁵ conjointement sont un alkylène ou un alcénylène, dans lequel éventuellement l'un des atomes de carbone peut être remplacé par O ou NH ;
ou un sel acceptable sur le plan cosmétique correspondant.

7. Utilisation cosmétique selon l'une quelconque des revendications précédentes, dans laquelle le composé de formule (I) ou (II) est un composé de formule
| | |
|---|---|
| (A) | 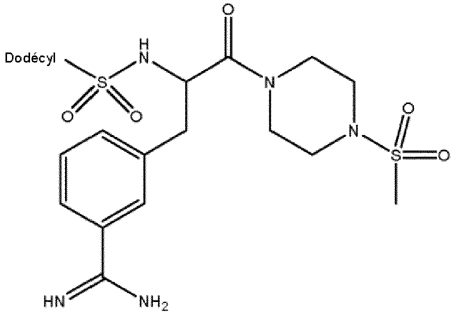 |
| (B) | 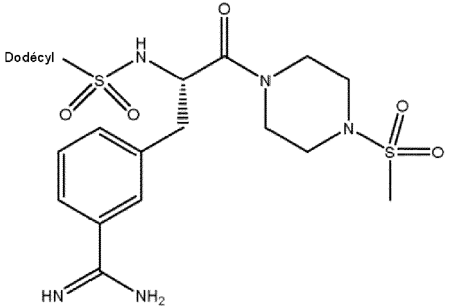 |
| (C) | 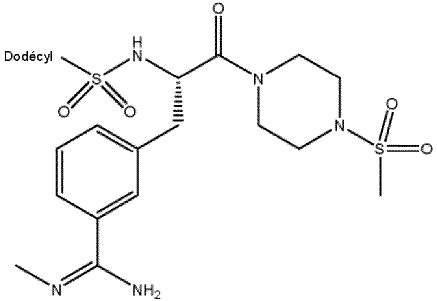 |
| (D) | 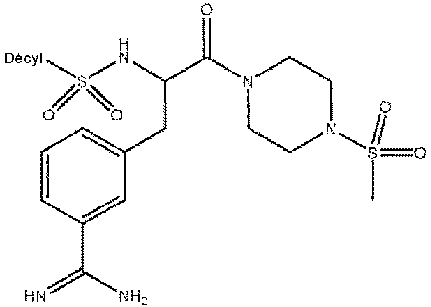 |
| (E) | 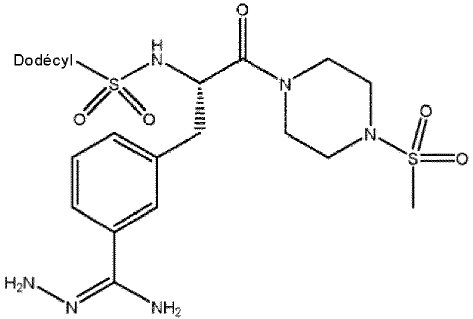 |
| (F) | 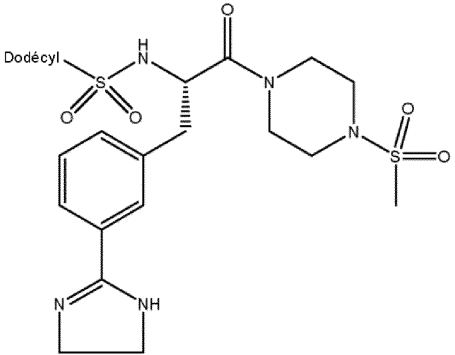 |
| (G) | 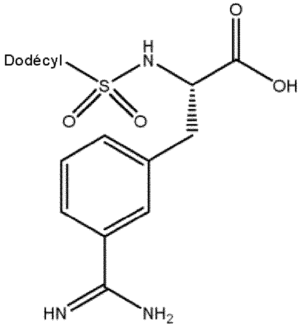 |
| (H) | 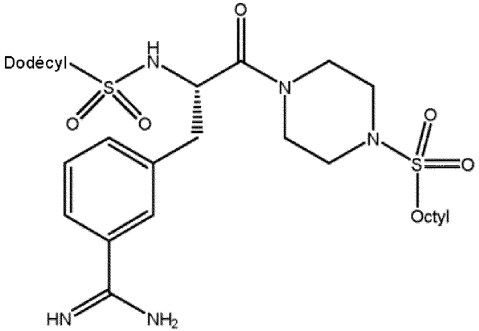 |
| (I) | 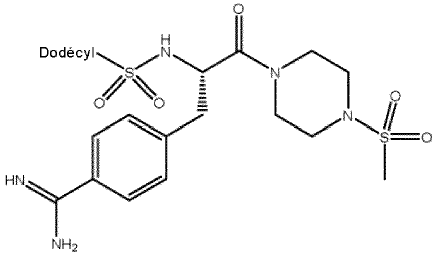 |
| (J) | 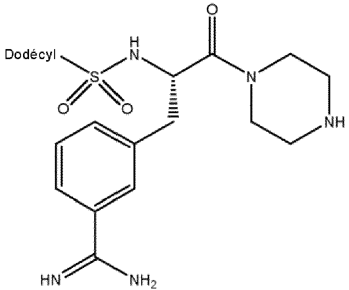 |
| (K) | 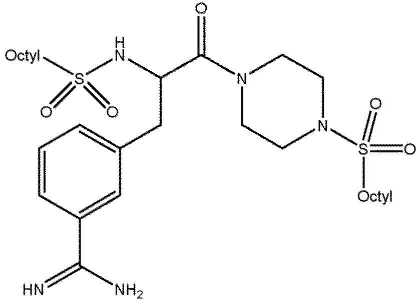 |
| (L) | 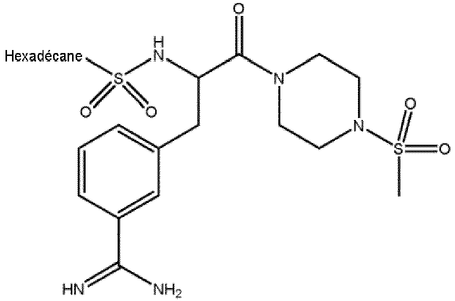 |
| (M) | 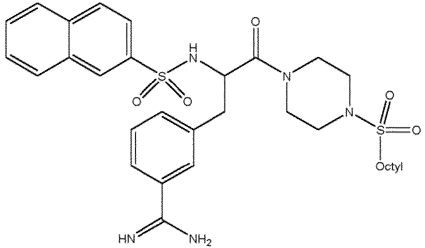 |
| (N) | 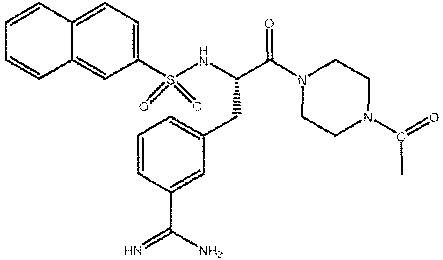 |
| (O) | 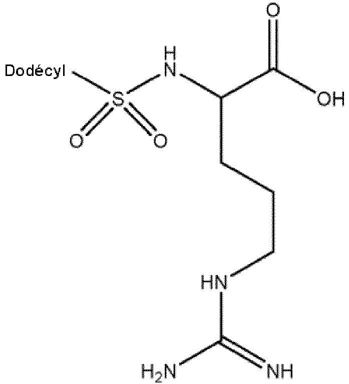 |
| (P) | 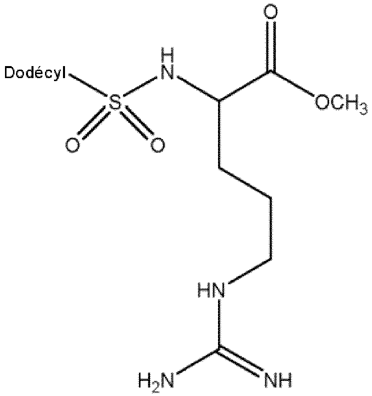 |
| (Q) | 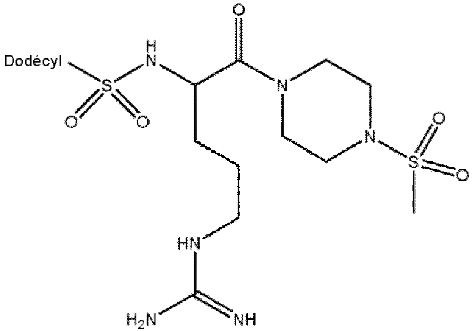 |
ou un sel acceptable sur le plan cosmétique correspondant.

8. Utilisation cosmétique selon l'une quelconque des revendications précédentes, dans laquelle la quantité du composé de formule (I) ou (II) ou des sels respectifs correspondants dans la composition est choisie dans la plage de 0,0001 à 5,0 % en poids, préférablement dans la plage de 0,001 à 1,0 % en poids, le plus préférablement dans la plage de 0,01 à 0,1 % en poids, sur la base du poids total de la composition.

9. Utilisation cosmétique selon l'une quelconque des revendications précédentes, dans laquelle la composition comprend en outre un rétinoïde, préférablement du rétinol.

10. Utilisation cosmétique selon la revendication 9, dans laquelle la quantité de rétinoïde est choisie dans la plage de 0,001 à 10 % en poids, préférablement d'environ 0,005 à 0,5 % en poids, le plus préférablement d'environ 0,03 à 0,3 % en poids, sur la base du poids total de la composition.

11. Utilisation cosmétique d'un composé de formule (I) ou (II) ou d'un sel acceptable sur le plan cosmétique correspondant tel que défini dans l'une quelconque des revendications 1 à 7 comme inhibiteur de LRAT (lécithine : rétinol acyltransférase).

12. Procédé pour augmenter l'efficacité du rétinol endogène en inhibant l'estérification du rétinol dans des kératinocytes, en particulier dans le traitement du vieillissement cutané, ledit procédé comprenant l'étape d'application par voie topique d'un composé de formule (I) ou (II) ou d'un sel acceptable sur le plan cosmétique correspondant tel que défini dans l'une quelconque des revendications 1 à 7 sur la peau d'une personne en ayant besoin, dans lequel l'efficacité est évaluée par rapport à un témoin non traité.

13. Procédé pour prévenir et/ou traiter le vieillissement cutané, préférablement pour lisser les rides et les ridules, pour diminuer leur volume et leur profondeur, pour traiter l'affaissement de la peau et/ou pour améliorer la fermeté de la peau chez une personne qui en a besoin, ledit procédé comprenant l'étape d'application par voie topique d'un composé tel que défini dans l'une quelconque des revendications 1 à 7 sur la zone affectée.

14. Composition topique de soin de la peau se présentant sous la forme d'une émulsion comprenant au moins un composé de formule (I) ou (II) 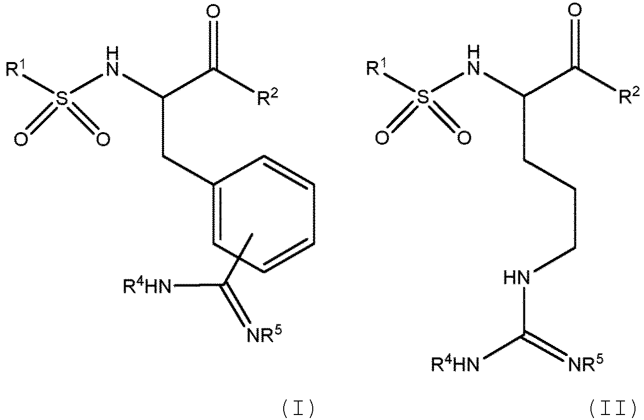 dans laquelle
R¹ est un groupe C₆-C₂₀alkyle ou un groupe naphtyle ;
R² est OH, SH, un groupe C₁-C₅alcoxy, un groupe C₁-C₅thioalcoxy ou un radical de formule (III) 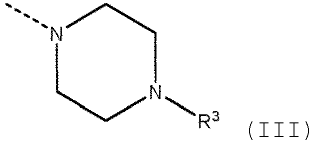
dans laquelle la ligne en pointillés représente la connexion au groupe carbonyle et R³ est choisi parmi H, un groupe C₁-C₁₅alkylsulfonyle ou un groupe C₁-C₅acyle ;
R⁴ est H ;
R⁵ est H, NH₂ ou un groupe C₁₋₅ alkyle ;
ou R⁴ et R⁵ conjointement sont un groupe alkylène ou un groupe alcénylène, dans lequel éventuellement l'un des atomes de carbone peut être remplacé par O ou NH ;
ou un sel acceptable sur le plan cosmétique correspondant et un support acceptable sur le plan cosmétique.

15. Composition cosmétique comprenant un rétinoïde et au moins un composé de formule (I) ou (II). 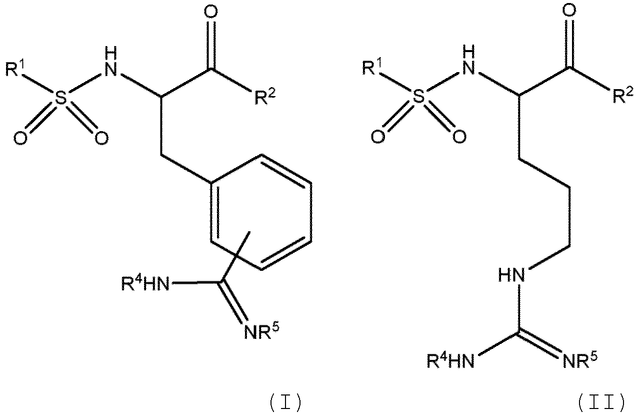 dans laquelle
R¹ est un groupe C₆-C₂₀alkyle ou un groupe aryle ;
R² est OH, SH, un groupe C₁-C₅alcoxy, un groupe C₁-C₅thioalcoxy ou un radical de formule (III) 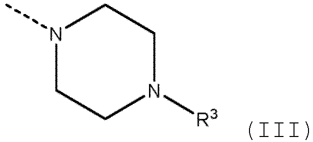
dans laquelle la ligne en pointillés représente la connexion au groupe carbonyle et R³ est choisi parmi H, un groupe C₁-C₁₅alkylsulfonyle ou un groupe C₁-C₅acyle ;
R⁴ est H ;
R⁵ est H, NH₂ ou un groupe C₁₋₅ alkyle ;
ou R⁴ et R⁵ conjointement sont un groupe alkylène ou un groupe alcénylène, dans lequel éventuellement l'un des atomes de carbone peut être remplacé par O ou NH ;
ou un sel acceptable sur le plan cosmétique correspondant et un support acceptable sur le plan cosmétique.
